# EUROPEAN PATENT APPLICATION

(11) **EP 2 915 883 A1**
(43) Date of publication of application: **09.09.2015**
(21) Application number: 14158403.7
(22) Date of filing: 07.03.2014
(51) Int. Cl.: C12Q 1/68

(54) **Non-invasive assay for early detection of cancer**

(71) Applicant: Ruprecht-Karls-Universität Heidelberg, 69117 Heidelberg (DE)
(72) Inventor: The designation of the inventor has not yet been filed
(74) Representative: Steinecke, Peter

(57) **Abstract**

Provided is a novel method for the early detection and/or discrimination of cancer and anticancer agents for the prevention or early treatment of cancer. In particular, the method relates to the status of methylation and/or expression of genes in a subject suspected to develop cancer or having cancer at an early stage. Furthermore, kits, devices, pharmaceutical compositions as well as methods related thereto are described.

SEQUENCE LISTING

<110>  Ruprecht-Karls-Universität Heidelberg

<120>  Non-invasive Assay for early Detection of Cancer

<130>  UN56A01/P-EP

<160>  12

<170>  PatentIn version 3.5

<210>  1
<211>  286
<212>  DNA
<213>  Artificial Sequence

<220>
<223>  S100P amplicon

<400>  1
ggaaggtggg tctgaatcta gcaccatgac ggaactagag acagccatgg gcatgatcat      60

agacgtcttt tcccgatatt cgggcagcga gggcagcacg cagaccctga ccaaggggga     120

gctcaaggtg ctgatggaga aggagctacc aggcttcctg caggtgagcc aggccggcag     180

tgctggactc agcgggggct ggggaagaag gggaaggcgt ggcaggcaga gggctgagag     240

ctgcggtggg gtcggcggtc aaggggctca gaggcaagag ggacag                    286


<210>  2
<211>  329
<212>  DNA
<213>  Artificial Sequence

<220>
<223>  SLC22A18 amplicon

<400>  2
caagtggaac cctggcaccc ctggacttcc tcctccagag gaagcggctt tggttgtggg      60

ccagcgagcc tgtgcgtccc cagccccagg gcattcaccg cttcagagaa gcaaggcgac     120

agttctgccg gatgagagga tccaggctga ctggaggaag gaagggcttt ggctcatctg     180

ggctcaggtt tgggagaggt ggcttctcag aggaggtgat gcctcagcca gtcttaaagg     240

tgagcacgga ttgtttgggt atgggaaggg catcctggct gagagaatag catagataac     300

ggcttagagg tgagcccagg cttggagtg                                       329


<210>  3
<211>  498
<212>  DNA
<213>  Artificial Sequence

<220>
<223>  DYRK4 amplicon

<400>  3

ggcctttcta aaactggctt tggactaaaa ccaggtcttt cacctgcctt ctcagggtcc 60

agtccgtctg cactcacctg atacagcagg gatgacttca gcaggacttt cttgctaatg 120

tgtgggaagc ttaccgtatt cttcttcccc ttggtgtcca caaaggggag tgaagtaaca 180

ctggtgttct gtagaaggag aaattgcatg tgttacaaaa gccttaggca agaaaacgtc 240

tccaggggtc atttcttcca gcctcccttc ccaattcccc aacaagaatg agcctaaccc 300

caatctggac ctaaacctat tcttaaagtt cttggcaaga aatgcagatt ccagagcagc 360

agagtcaaca aacacatggg ctctgggttt gagtccctgc tctactactt actagctgca 420

ggctttcaac agctcacact gactctttct cgaaacctca attatttcat cactaccatg 480

ggaacaaaaa tggggcct 498

<210> 4
<211> 392
<212> DNA
<213> Artificial Sequence

<220>
<223> FUT7 amplicon

<400> 4
gaagaggaag ggatccagcc tgaagtccag actccccgct ccctctaagc cagggcctgg 60

agcctggagg ccaggttcct tcttctacac cagcccacgt tgggtgccag ccaggctggg 120

atggccctgc ggggtcaccc tgagccccag ccaaccaaca ccccactctc agccacagtg 180

ggaggcccca tcagcctctt cacccaacca cgttgccact ctgctgcacg ggaccttgtg 240

tggtcccagg cgtggccaga cccagacgtc ctggagatct caatgcaggt caggcttggt 300

ttgtttgttt gtttgtttgt ttgttttttg agagggagtc ttgcactatc gcccaggctg 360

taatcccagc attttgggag gccaaggctt gt 392

<210> 5
<211> 35
<212> DNA
<213> Artificial Sequence

<220>
<223> S100P Primer sense

<400> 5
aggaagagag ggaaggtggg tttgaattta gtatt 35

<210> 6
<211> 56
<212> DNA
<213> Artificial Sequence

<220>
<223> S100P Primer antisense

<400>  6
cagtaatacg actcactata gggagaaggc tctatccctc ttacctctaa acccct          56

<210>  7
<211>  35
<212>  DNA
<213>  Artificial Sequence

<220>
<223>  DYRK4 Primer sense

<400>  7
aggaagagag ggttttttta aaattggttt tggat                                 35

<210>  8
<211>  55
<212>  DNA
<213>  Artificial Sequence

<220>
<223>  DYRK Primer antisense

<400>  8
cagtaatacg actcactata gggagaaggc taaaccccat ttttattccc ataat           55

<210>  9
<211>  35
<212>  DNA
<213>  Artificial Sequence

<220>
<223>  FUT7 Primer sense

<400>  9
aggaagagag gaagaggaag ggatttagtt tgaag                                 35

<210>  10
<211>  56
<212>  DNA
<213>  Artificial Sequence

<220>
<223>  FUT7 Primer antisense

<400>  10
cagtaatacg actcactata gggagaaggc tacaaacctt aacctcccaa aatact          56

<210>  11
<211>  35
<212>  DNA
<213>  Artificial Sequence

<220>
<223>  SLC22A18 Primer sense

<400>  11
aggaagagag taagtggaat tttggtattt ttgga                                 35

<210>  12
<211>  56
<212>  DNA
<213>  Artificial Sequence

<220>
<223>  SLC22A18 Primer antisense

<400>  12
cagtaatacg actcactata gggagaaggc tcactccaaa cctaaactca cctcta          56

## Description

### Field of the invention

The present invention relates to methods for the early detection of cancer and to anti-cancer agents for the prevention or early treatment of cancer. In particular, the present invention provides methylation and expression panel of four genes in peripheral blood as a marker for the early detection of breast cancer, ovarian cancer, and pancreatic cancer as well as anti-cancer prophylactic and early treatment regimens based on the status of methylation and/or expression of said genes in a subject suspected to develop cancer or having cancer at an early stage.

### Background of the invention

Cancer is one of the most important medical and health problems in the world. As the leading cause of death worldwide, there were 12.4 million new cancer cases and 7.6 million cancer related deaths in 2008¹. It has been predicted that the deaths from cancer worldwide will continuously rise and 12 million deaths would be caused by cancer in the year of 2030¹. Breast cancer (BC) is the most common cancer and the leading cause of cancer-related mortality among women², with 1.38 million new cases and 458,000 deaths in 2008³. Although therapeutic advances have improved the survival rate of BC patients, most BC patients still suffer from greatly reduced quality of life or even metastasis due to delayed diagnosis^{2,4}. Compared to BC, ovarian cancer (OvCa) is comparable rare in occurrence, but is the leading cause of death from gynecologic cancers because of its high malignancy⁵. In 2008, 225,000 women were diagnosed with OvCa worldwide, and 140,000 of these women died from the disease⁶. Typically, women with the OvCa present only a few early symptoms, and thus nearly three-quarters of ovarian cancer cases are presented at an advanced stage, where the disease spread well beyond the ovaries⁷. Pancreatic cancer (PaCa) is the most aggressive of all epithelial malignancies⁸. With 279,000 new diagnoses of PaCa worldwide, the 5-year overall survival rate of PaCa patients is less than 5%^{9,10}. Although recent genome-wide association studies (GWAS) have successfully detected several genetic variants associated with the risk of BC, OvCa, and PaCa, no valuable marker for the early detection of BC has been identified. As an early event in the development of cancer, changes in epigenetics, e.g. of DNA methylation are particularly promising as markers for the early detection of cancer¹¹⁻¹³. Recent studies have shown that methylation analysis of blood cell DNA can serve as a reliable and robust marker^{14,15}. Intensive studies have disclosed altered DNA methylation signatures in cancer on the somatic level^{12,16-19}, whereas only a few studies with candidate-gene-approach have analyzed methylation signatures in peripheral blood DNA in cancer²⁰⁻²⁴. Marsit and co-workers have successfully identified bladder cancer associated DNA methylation in peripheral blood by Infinium 27K Array²⁵.

However, for several types of cancer, in particular BC, OvCa, and PaCa non-invasive techniques for the early detection are still missing. In addition, it would be highly desirable to have means for determining the susceptibility and risk evaluation for a subject to develop those cancers and to provide a non-invasive, highly efficient, easy, reliable, and low cost method for the early detection of BC, OvCa, and PaCa as well as other cancers. This technical problem has been solved by the embodiments as characterized in the claims and described further below and illustrated in the Examples.

### Summary of the invention

The present invention generally relates to a non-invasive method for early diagnosing cancer or assertive possibility of developing cancer in a subject comprising the detection of the methylation and expression profile of a panel of novel biomarkers. As shown in the Examples, the present invention is based on the observation that differentially methylated genomic CpG nucleotide sequences and expression level of genes are associated with cancer, in particular breast cancer (BC), ovarian cancer (OvCa), and pancreatic cancer (PaCa). As disclosed herein, the methods of the invention have numerous diagnostic, prognostic, and therapeutic applications.

Thus, in one aspect the present invention relates to a non-invasive method comprising (a) providing a DNA, RNA, and/or protein sample from the subject; and (b) determining the methylation status and/or the expression level of one or more genes or proteins selected from the group consisting of S100P (S100 calcium binding protein P), SLC22A18 (solute carrier family 22, member 18), DYRK4 (dual-specificity tyrosine-(Y)-phosphorylation regulated kinase 4) and FUT7 (fucosyltransferase 7).

An altered, preferably a decreased methylation and/or over-expression is compared to a corresponding reference value or control sample of a healthy subject which is indicative for the presence or risk and susceptibility of cancer development.

In a particularly preferred embodiment the methylation comprises methylation of at least one CpG site and/or the determination of the expression level by measuring the mRNA and/or protein level.

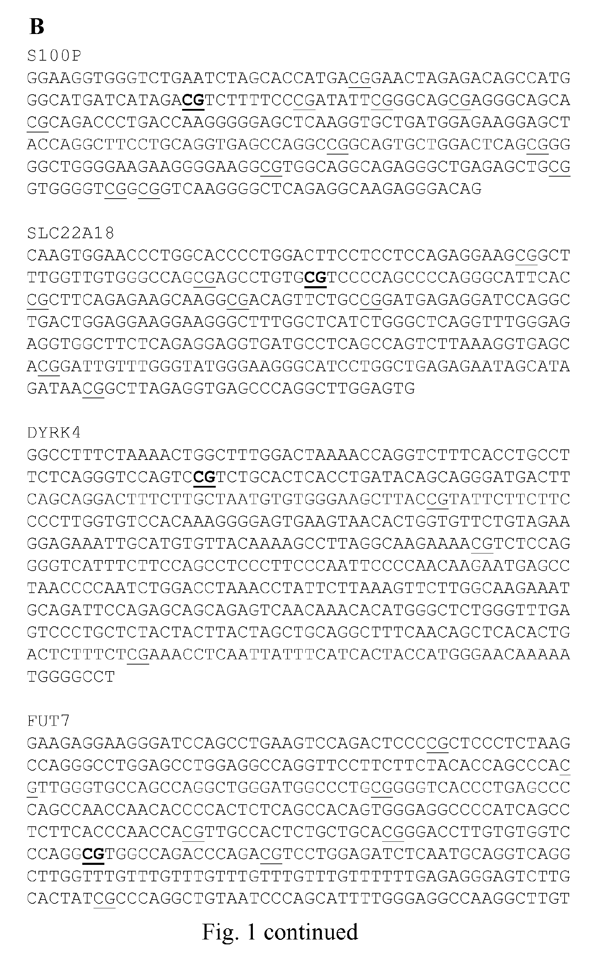

In one preferred embodiment, at least the methylation of one CpG site as shown in Fig. 1B is determined. Preferably, at least the methylation of one or more CpG sites is determined selected from the group consisting of CpG sites cg22266967 in S100P (Chr 4: 6746599, Build 36.1/hg18), cg21019522 in SLC22A18 (Chr 11: 2877365, Build 36.1/hg18), cg09418321 in DYRK4 (Chr 12: 4569879, Build 36.1/hg18) and cg02679745 in FUT7 (Chr 9: 139047467, Build 36.1/hg18).

In one embodiment the method comprises determining the methylation status of at least 2, at least 3, at least 4, at least 5, at least 6 or at least 7 of the CpG sites of said CpG sites. In a preferred embodiment, the CpG sites of at least 2, at least 3 or at least 4 of the genes are determined.

In a further embodiment, the DNA methylation is determined by using bisulfite-specific primers, preferably selected from the group of primers amplifying the nucleotide sequence of S100P (NC_000004.12 Reference GRCh38 Primary Assembly, AC_000136.1 Alternate HuRef, NC_018915.2 Alternate CHM1_1.1), SLC22A18 (NC_000011.10 Reference GRCh38 Primary Assembly, NG_011512.1 RefSeqGene, NT_187585.1 Reference GRCh38 ALT_REF_LOCI_1, AC_000143.1 Alternate HuRef, NC_018922.2 Alternate CHM1_1.1), DYRK4 (NC_000012.12 Reference GRCh38 Primary Assembly, AC_000144.1 Alternate HuRef, N_018923.2 Alternate CHM1_1.1), FUT 7 (NC_000009.12 Reference GRCh38 Primary Assembly, NG_007527.1 RefSeqGene,AC_000141.1 Alternate HuRef, NC_018920.2 Alternate CHM1_1.1) or its bisulfite-converted sequence. Preferably, the primer or primer pair is selected from the group of primers comprising or consisting of the nucleotide sequence of SEQ ID NOs 5 and 6, 7 and 8, 9 and 10, and 11 and 12, respectively.

In one aspect the sample from the subject provided by the method of the present invention comprises peripheral blood. Due to the sensitivity and reliability of the novel biomarkers the method of the present invention advantageously can be applied to subjects which are substantially healthy and do not show any significant symptoms or signs of cancer. Furthermore, the experiments performed in accordance with the present invention revealed that cancer, in particular BC can be diagnosed in women at relative early age. Thus, in one embodiment the age of the subject is < 50.

Most cancers develop over time due to genetic damage, where no identifiable inherited gene involved and are therefore considered to be sporadic. In contrast, familial cancers tend to occur at a younger age than sporadic cancers and are believed to be due to a combination of risk factors which include inherited susceptibility, environmental factors, and chance. Since a diagnosis of cancer at an early stage was shown in experiments performed in accordance with the present invention, in one embodiment the subject is a familiar cancer patient.

The alteration in methylation status and expression level in the candidate genes has been shown in accordance with the present invention to be affected in subjects suffering from breast cancer (BC), ovarian cancer (OvCa), or pancreatic cancer (PaCa). Thus, in one embodiment the cancer which is to be determined, diagnosed, ameliorated, and/or treated is breast cancer (BC), ovarian cancer (OvCa), or pancreatic cancer (PaCa).

In another embodiment of the present invention, the cancer is discriminated by the methylation level in specific CpG loci.

In one embodiment a kit for use in the method of the present invention is designed. The kit comprises one or more primers for amplifying a fragment comprising a CpG site and/or for analyzing the product amplified by a primer pair, wherein said kit comprises instructions for carrying out said method, and optionally standards for a control or reference. In a preferred embodiment, the primer pair is a primer pair amplifying the nucleotide sequence of S100P (NC_000004.12 Reference GRCh38 Primary Assembly, AC_000136.1 Alternate HuRef, NC_018915.2 Alternate CHM1_1.1), SLC22A18 (NC_000011.10 Reference GRCh38 Primary Assembly, NG_011512.1 RefSeqGene, NT_187585.1 Reference GRCh38 ALT_REF_LOCI_1, AC_000143.1 Alternate HuRef, NC_018922.2 Alternate CHM1_1.1), DYRK4 (NC_000012.12 Reference GRCh38 Primary Assembly, AC_000144.1 Alternate HuRef, N_018923.2 Alternate CHM1_1.1), FUT 7 (NC_000009.12 Reference GRCh38 Primary Assembly, NG_007527.1 RefSeqGene, AC_000141.1 Alternate HuRef, NC_018920.2 Alternate CHM1_1.1) or its bisulfite-converted sequence. Preferably, the primer or primer pair is selected from the group of primers comprising or consisting of the nucleotide sequence of SEQ ID NOs 5 and 6, 7 and 8, 9 and 10, and 11 and 12, respectively.

In one embodiment, the present invention relates to a device for diagnosing cancer, comprising (a) an analyzing unit comprising (i) a detection agent for determining the methylation status and/or expression level as defined in the non-invasive method of the present invention as described alone in a sample of a subject; and preferably (ii) receptacle for a sample of a subject suspected to suffer from or being at risk of developing cancer; and (b) an evaluation unit comprising a data processor having tangibly embedded an algorithm for carrying out a comparison of the amount determined by the analyzing unit with a stored reference and which is capable of generating an output file containing a diagnosis established based on the said comparison.

Furthermore, in one embodiment the present invention relates to a method comprising the steps of: (a) providing a sample from the subject being treated; and (b) determining the methylation status and/or expression level as defined in the non-invasive method for early diagnosing cancer or a susceptibility of developing cancer in a subject, wherein an altered methylation and/or expression, preferably an increase of methylation and/or decrease of expression compared to a reference sample obtained from the subject before or at an earlier stage of the treatment indicate effectiveness of a treatment course undergoing by the subject.

In one embodiment the present invention is further directed to an anti-cancer agent for use in the prevention, amelioration or treatment of cancer in a patient at an early stage or before the onset of the cancer, wherein the patient is characterized by an altered methylation status and/or expression, preferably a decreased methylation status and/or increased expression of one or more genes and/or proteins selected from the group consisting of S100P, SLC22A18, DYRK4 and FUT7 as compared to a healthy subject or reference value. In a preferred embodiment the patient has been diagnosed by the method for early diagnosing cancer or a susceptibility of developing cancer in a subject. In a particularly preferred embodiment (i) the cancer is BC and (ii) the patient is younger than 50 years and/or does not show micro-metastasis in lymph nodes.

In another embodiment the dose of the anti-cancer agent for use in the prevention, amelioration or treatment of a cancer in a patient is adjusted in accordance with (i) the diagnosis of the method for early diagnosing cancer or a susceptibility of developing cancer in a subject; or (ii) the progression or regression of the cancer as assessed by the method for monitoring the therapy of a subject being treated against cancer.

The anti-cancer agent may be selected from the group consisting of, but not limited to Herceptin (Trastuzumab), Avastin (Bevacizumab), and the thyrosine kinase inhibitiors such as Lapatinib, Sorafinib oder Sunitini; Epirubicin, Doxorubicin, Cyclophosphamid, Taxol, Taxotere, Carboplatin, GnRH-analoga, estrogen receptor blocker such as Tamoxifen and Fulvestrant, aromatase inhibitors such as Arimidex, Femara, and Aromasin; Perjeta (Pertuzumab).

Furthermore, in one embodiment the present invention relates to a method of classifying cancer or determining the risk of developing cancer in a subject, comprising: (a) providing a sample from a subject, said sample comprising genomic DNA; mRNA, and/or protein (b) detecting the presence or absence of DNA methylation and/or expression level in one or more genes or proteins to generate a methylation profile and/or expression profile for said subject, wherein the one or more genes is selected from the group consisting of S100P (NM_005980.2, S100 calcium binding protein P), SLC22A18 (NM_002555.5 variant 1, NM_183233.2 variant 2, Solute carrier family 22, member 18), DYRK4 (NM_003845.2 variant 1, NM_001282285.1 variant 2, NM_001282286.1 variant 3, and NR_104115.1 variant 4, dual-specificity tyrosine-(Y)-phosphorylation regulated kinase 4) and FUT7 (NM_004479.3, fucosyltransferase 7), and optionally HYAL2 gene (NM_003773.4 variant1, NM_033158.4 variant 2, hyaluronoglucosaminidase 2); and (c) comparing said methylation profile and/or expression profile to one or more standard methylation profiles and/or expression profiles, wherein said standard methylation profiles and/or expression profiles are selected from the group consisting of methylation profiles and/or expression profiles of non-cancerous samples and methylation profiles and/or expression profiles of cancerous samples.

As an advantage of the method of the present invention, it has been shown that an altered methylation profile and/or expression status can be measured in peripheral blood of the subjects. Thus, in one embodiment the sample from the subject is a body fluid, preferably a sample of peripheral blood or derived thereof.

In a further embodiment the present invention is directed to a non-transient computer readable storage medium, comprising executable instructions for detecting cancer or a susceptibility of developing cancer, the executable instructions configured to (a) receive data characterizing the intensity of DNA methylation and/or expression profile in one or more gene loci of the candidate genes; and (b) compare the intensity of DNA methylation and/or expression profile to a model; and calculate a likelihood of cancer.

In one embodiment, the present invention relates to a method of dosing an anti-cancer agent or an agent, as described above for use in amelioration of symptoms associated with the development of cancer in a subject comprising the method for early diagnosing cancer or a susceptibility of developing cancer described herein and preparing a pharmaceutical composition comprising an effective amount of and/or dosage regime for use of the agent in the treatment of the subject. Thus, in this embodiment advantage is taken of the early diagnosis of cancer, in particular breast cancer in that similar to microarray gene expression profiling an integrated approach for tailored treatment in cancer such as breast cancer is feasible; see, e.g., for review Awada and de Castro Jr. in Annals of Oncology 16 (Supplement 2) (2005), ii203-ii208. For example, a subject may be diagnosed to be at risk of developing cancer in accordance with the method of the present invention because of a decreased methylation status and/or increased expression level of any one of the genes S100P, SLC22A18, DYRK, and FUT7, optionally diagnosed for and confirmed by further risk indicators. In this case, an anti-cancer agent or other anti-cancer treatment can be formulated at low dose such as to prevent the onset of the disease on the one hand and avoid side effects due to the treatment on the other. Similarly, if in the course of the cancer treatment the methylation status and/or expression level of any one of the mentioned genes increased and decreased, respectively, this may be indicative for the responsiveness of the patient to treatment and allows reducing the dose of treatment for the benefit of the patients comfort.

Hence, it is a particular object of the present invention to use of one or more genes selected from the group consisting of S100P, SLC22A18, DYRK, and FUT7 as a biomarker for the early detection and/or classification of cancer.

Further embodiments of the present invention will be apparent from the description and Examples below.

The foregoing description of related art is not intended in any way as an admission that any of the documents described therein are prior art to embodiments of the present invention. Each of the publications cited therein, e.g., journal articles, patent applications and GENEBANK Accession numbers, is incorporated by reference herein.

### Brief description of the drawings

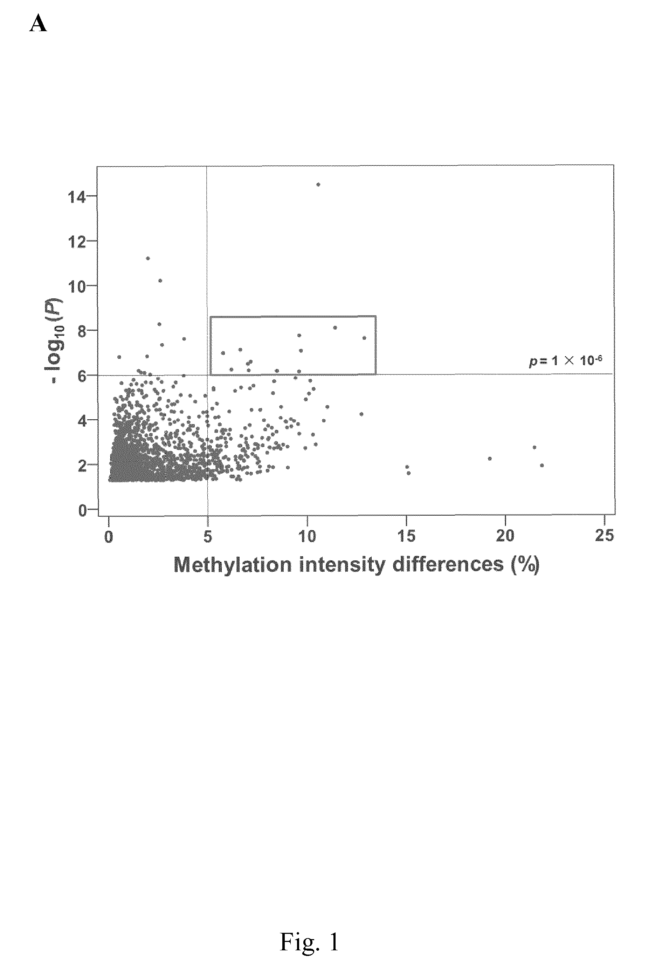

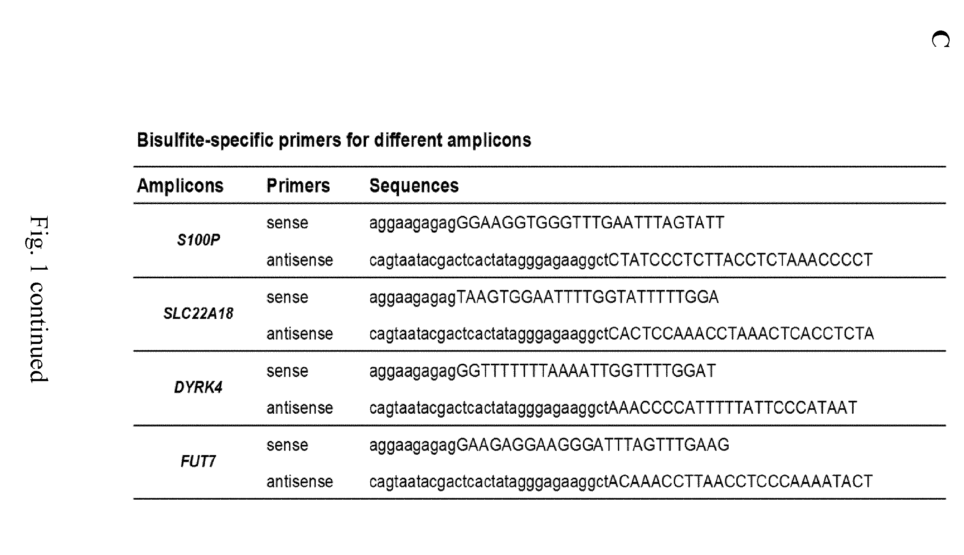

**Fig. 1**(A) Identification of most promising BC-associated methylation sites in the peripheral blood DNA by Illumina 27K Array in the discovery round. Significant 2,125 BC-associated methylation loci identified in the discovery round by Illumina 27K Array are presented as -log₁₀(*P*) versus absolute methylation intensity difference between familial BC cases and controls (the P-value is false-discovery-rate adjusted, p < 0.05). The 12 most promising BC-associated methylation sites (difference > 5%, *p* < 1.0 × 10⁻⁶) are in the orange [grey] box. The two lines indicate the threshold of adjusted *p* = 1.0 × 10⁻⁶ and methylation intensity difference = 5%, respectively.
   Amplicon and primer design for methylation analysis. (B) Sequences of amplicons in S100P, SLC22A18, DYRK4 and FUT7 genes examined by MassARRAY. In all four CpG amplicons, the methylation levels of 19 CpGs sites in 18 distinguishable peaks could be determined the MassARRAY assay. The CpG sites are underlined. The measurable CpG sites were underlined and in grey. The CpG sites measured by Illumina 27K, cg22266967 in S100P, cg21019522 in SLC22A18, cg09418321 in DYRK4, cg02679745 in FUT7, are underlined, in grey and bold. (C) The list of bisulfite-specific primers for the four amplicons. Uppercase letters indicate the sequence specific primer regions, and non-specific tags are shown in lower case letters. There are no SNPs located in the primer regions, and none of the measurable 19 CpGs in the four amplicons are overlapped with known SNPs.

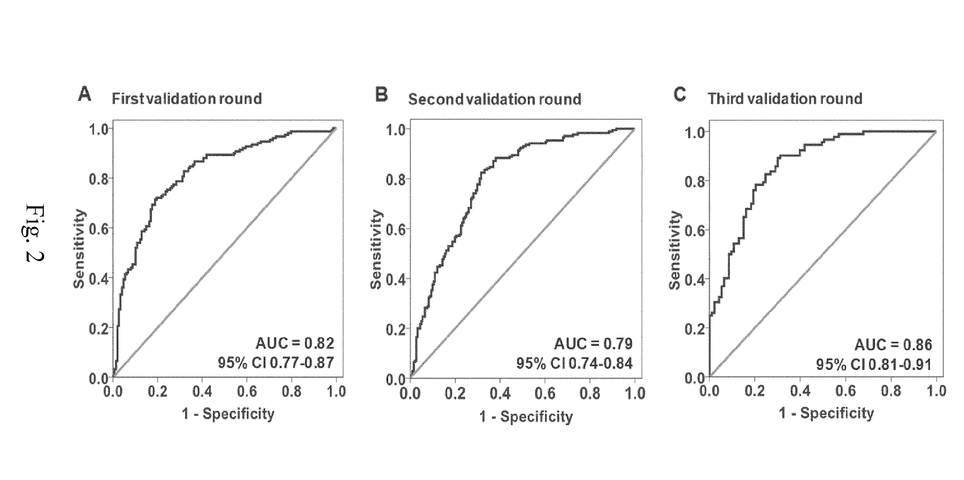

**Fig. 2****:** Blood-based methylation panel and early BC detection. (A-C) Logistic regression model based ROC curve analysis for the discriminatory power of methylation panel of four genes (S100P, SLC22A18, DYRK4, and FUT7) to distinguish BC cases from healthy controls in three validation rounds. The light blue line represents the line of no-discrimination.

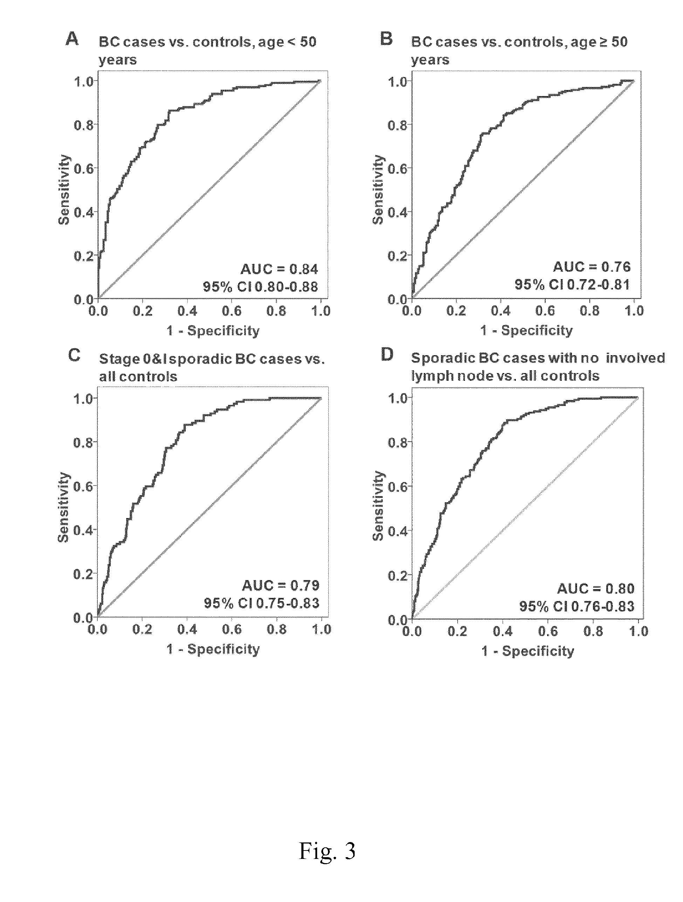

**Fig. 3****:** Blood-based methylation panel and BC detection in subgroups. (A-B) Logistic regression model based ROC curve analysis for the discriminatory power of methylation panel of four genes (S100P, SLC22A18, DYRK4, and FUT7) to distinguish BC cases from healthy controls in different age groups (C) The discrimination of early stage sporadic BC from controls by methylation panel. (D) The discrimination of sporadic BC patients with no involved lymph node from controls by methylation panel. The light blue line represents the line of no-discrimination.

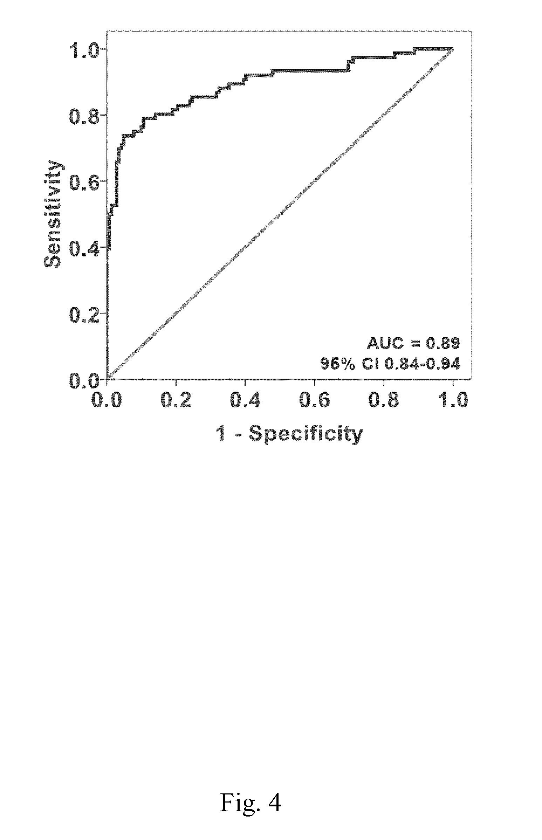

**Fig. 4****:** Methylation panel and the early detection of OvCa. Logistic regression model based ROC curve analysis for the discriminatory power of methylation panel of S100P, SLC22A18 and FUT 7 to distinguish OvCa cases from healthy controls. The light blue line represents the line of no-discrimination.

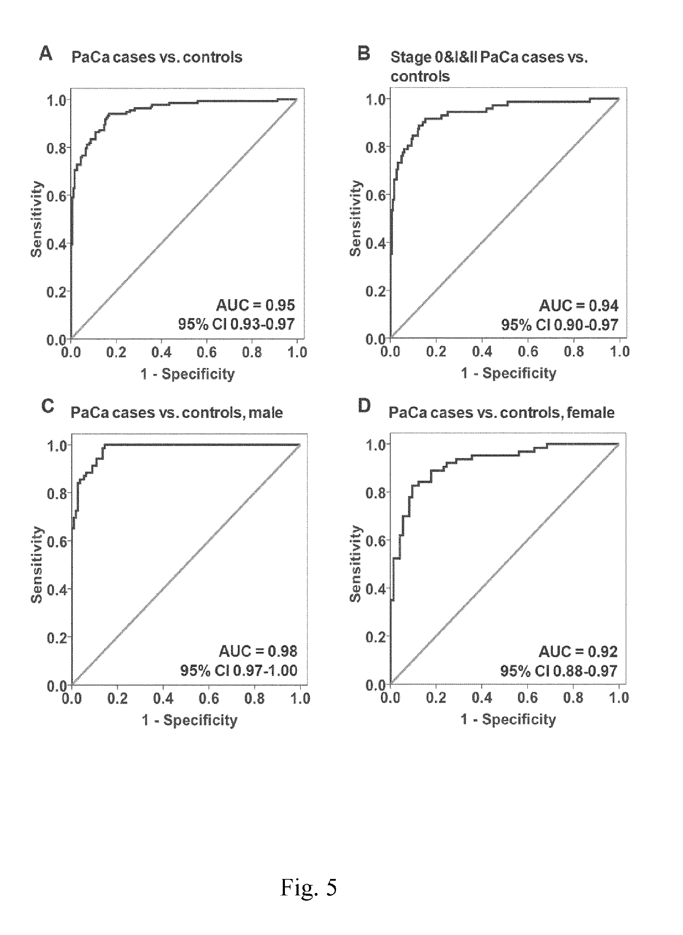

**Fig. 5****:** Blood-based S100P methylation and the detection of PaCa. (A) Logistic regression model based ROC curve analysis for the discriminatory power of methylation panel of S100P to distinguish sporadic PaCa cases from healthy controls. **(B)** The discrimination of early stage sporadic PaCa cases from controls by S100P methylation panel. **(C-D)** The gender-related discriminatory power of distinguishing sporadic PaCa patients from controls by S100P methylation panel. The light blue line represents the line of no-discrimination.

### Detailed description of the invention

The present invention generally relates to a method for early diagnosing cancer or a susceptibility of developing cancer. As shown in the Examples epigenetic changes in the form of changes in the methylation status of genomic DNA isolated from peripheral blood could be associated with the malignancy at early stage. Therefore, the present invention provides cancer DNA methylation signatures in peripheral blood which could serve as a marker for the early detection of cancer, in particular breast cancer (BC), ovarian cancer (OvCa), and pancreatic cancer (PaCa). More specifically, the present invention relates to a non-invasive method for early diagnosing cancer or a susceptibility of developing cancer in a subject comprising
(a) providing a biological sample from the subject, preferably a blood sample which may be further processed to isolate and contain at least DNA, i.e. genomic DNA; RNA, *i.e.* mRNA, and/or protein; and
(b) determining (i) genomic modifications, i. e. chemical modifications, in particular the methylation status and/or (ii) the expression level of one or more candidate genes selected from the group consisting of S100P (S100 calcium binding protein P), SLC22A18 (Solute carrier family 22, member 18), DYRK4 (dual-specificity tyrosine-(Y)-phosphorylation regulated kinase 4), and FUT7 (fucosyltransferase 7);
wherein the alterations compared to a control sample obtained from a healthy subject or to a corresponding reference value is indicative for the presence or risk and susceptibility of developing cancer, in particular in case of a decreased methylation and/or increased expression level of any of the candidate genes.

As illustrated in the appended Examples, the method of the present invention at first place is based on observations on epigenetic changes, *i.e.* the decreased methylation status of the mentioned genes with confirmation of an inverse relationship on the level of their expression. Accordingly, while the method of the present invention will be mainly explained by way of reference to the methylation status of the marker genes the description is meant to apply to the corresponding embodiments with an altered expression level as well; see also the summary of the invention above.

In this context, without intending to be bound by theory in accordance with the present invention it is believed that genomic modifications, including epigenetic changes which do not involve a change in the DNA sequence, in particular a decreased methylation status of the candidate genes are reflective of both (i) the general methylation status of the genome in the subject probably also affecting other genes and genomic sequences associated with or even involved in cancer development, and (ii) the activity of the candidate gene on the level of expression.

This is because the altered methylation status of the genes compared to genomic DNA from healthy volunteers does not only occur in the promoter region which could explain the increased level of gene expression. However, hypermethylation or hypomethylation can also be observed within coding region (intragenic) or within non-coding region or between annotated genes (intergenic) as shown in the case of S100P where an altered methylation status could be almost always determined in CpG sites located at first exon. Also in DYRK4 at least one differentially methylated CpG site has been determined in the intragenic region.

Therefore, in accordance with the present invention the term "gene" as used herein includes cis-acting genomic DNA sequences comprising up- and downstream regulatory regions like 3'UTR, promoter, 5'UTR, silencers, enhancers, insulators, CCAAT box, TATA box, Locus Control Region (LCR), etc. located up to about 100 kb up- or downstream of the "gene body", i.e. promoter and exon/intron sequences. In a preferred embodiment, the up- and downstream sequences are located 50 kb, more preferably between about 20 kb and 10 kb away from the gene body, particular preferred about 5 kb or closer. However, such regulatory regions, in particular CpG methylation sensitive sequences may also be located more distantly and also include trans-acting sequences, for example which are located on another chromosome but being capable of influencing the methylation and expression level, respectively, of the subject gene. In addition, the term "gene" also comprises regions between the exon, *i.e.* non-coding regions or between annotated genes (intergenic).

In accordance with the present invention, preferably the altered methylation status of the 5th carbon of a cytosine base (5-methylcytosine) of CpG dinucleotides of the candidate genes was determined. However, methylation can also be observed at CpA, CpT, and CpC sequences and as 5-hydroxymethylcytosine. These methylation profiles can also be determined for the presence or risk and susceptibility of developing cancer, since their methylation might alter the expression level of any of the candidate genes.

Although changes in DNA methylation have been recognized as common molecular alternations in cancer, prior to the present invention body fluid based methylation changes associated with BC, OvCa, and/or PaCa had rarely been evaluated. Risk prediction and early detection are of great importance for better survival of cancer patients. However, no satisfactory marker for the early detection of cancer has been discovered³². The widely used blood-based BC markers, like CA15-3 and CA27-29, have poor sensitivity to early stage BC^{32,33}. For example, CA 15-3 concentrations are increased in about 10% of patients with stage I disease, 20% with stage II disease, 40% with stage III disease, and 75% with stage IV disease¹. Although mammography has been considered as an efficient tool for BC diagnosis, the concern of radiological exposure and the lack of sensitivity (sensitivity < 70%) to the high-dense breast in younger women have limited its utility³⁴. The blood level of CA-125 has been applied as a monitor for OvCa. However, is application of CA-125 for the early detection of OvCa has been limited to the advanced-stage OvCa, because the protein is elevated in less than 50% of stage I ovarian cancers⁵. As one of the most malignant cancer, about 90% PaCa cases were diagnosed at advanced stage, without efficient maker for its early detection⁹. Since the change in methylation occurs at an early event in the development of cancer, changes of DNA methylation are potential markers for the early detection of cancer¹¹⁻¹³. Moreover, the stability of DNA methylation serves a great advantage for a marker³⁵. Therefore, the present studies not only characterize DNA methylation changes associated with BC, OvCa, and/or PaCa, but also indicate that differential DNA methylation status may serve as diagnostic markers and/or targets for therapy for BC, OvCa, and/or PaCa.

In accordance with the present invention in the discovery round, a genome-wide investigation using Illumina 27K Methylation Array was performed (discovery round, 72 familial BC case and 24 controls) followed by a MassARRAY to validate promising CpG sites from three independent case-control studies on BC and two case-control studies on OvCa and PaCa utilizing peripheral blood.

As a surprising result, a panel of decreased methylation in four genes, i.e. S100P, SLC22A18, DYRK4 and FUT7 was identified to be associated with breast cancer (the methylation profile of the four genes, first validation round, 160 familial BC case and 160 controls: p = 1.58 × 10⁻¹⁷; second validation round, 189 sporadic BC case and 189 controls: p = 4.50 × 10⁻¹⁸; third validation round, 95 sporadic BC case and 95 controls: p = 3.05 × 10⁻¹⁸) and showed a remarkable power to distinguish early stage BC cases from healthy controls, as well as enabled outstanding power to detect BC in younger women as a tool independent from mammography. Moreover, the methylation panel had sufficient sensitivity to breast tumors with various clinical characteristics. The genes showed a decreased methylation within promoter region, the intragenic, or intergenic regions.

It is to be noted that the term "a" or "an" entity refers to one or more of that entity; for example, "a gene" is understood to represent one or more genes. As such, the terms "a" (or "an"), "one or more" and "at least one" can be used interchangeably herein. As used herein, the terms "early diagnosing cancer" or "early detection of cancer" refers to the assessment of the probability of developing cancer prior to metastasis. Preferably the probability is assessed before a morphological change of cells or tissues is observed.

S100P (NM_005980.2, S100 calcium binding protein P), also known as MIG9 (Migration-Inducing Gene 9 Protein), which belongs to the family of proteins containing 2 EF-hand calcium-binding motifs and is involved in the regulation of a number of cellular processes such as cell cycle progression and differentiation was one of the genes showing a decreased methylation in peripheral blood of patients affected with cancer. The CpG sites, wherein an altered methylation in peripheral blood could be observed, where located at the first exon of S100P. In particular, the CpG site cg22266967 of S100P (Chr 4: 6746599, Build 36.1/hg 18) showed a significant decrease in methylation. However, all other flanking CpG sites showed also a significant lower methylation status in 444 BC cases compared to healthy controls (n= 444).

In addition, SLC22A18 (NM_002555.5 variant 1, NM_183233.2 variant 2, Solute carrier family 22, member 18), also known as SLC22A1L (Solute carrier family 22 (organic cation transporter), member 1-like), BWSCR1A (Beckwith-Wiedemann syndrome chromosome region 1, candidate A), BWR1A (Beckwith-Wiedemann region 1A), IMPT1 (Imprinted polyspecific membrane transporter 1), ORCTL2 (Organic-cation transporter-like 2), HET, ITM, p45-BWR1A (P45 Beckwith-Wiedemann Region 1A) and TSSC5 (Tumor-suppressing subchromosomal transferable fragment candidate gene 5) which is located in an important tumor-suppressor gene region showed a decreased methylation status in its promoter region. Two alternatively spliced transcript variants encoding the same protein have been described, i.e. NM_002555.5 variant 1 and NM_183233.2 variant 2, however, a decrease in methylation could be observed in both variants in peripheral blood of patients affected with cancer. CpG site cg211019522 in SLC22A18 (Chr 11: 2877365, Build 36.1/hg 18) showed a significant decrease in methylation compared to the other CpG sites and healthy controls. However, a decrease in methylation status could also be observed in the accompanying CpG sites at SLC22A18.

Furthermore, a decreased methylation in peripheral blood could be observed in several transcript variants of DYRK4 (dual-specificity tyrosine-(Y)-phosphorylation regulated kinase 4), a member of the Dual-specificity tyrosine phosphorylation-regulated kinase (DYRK) family. The variants are generated due to alternative splicing, i.e. NM_003845.2 variant 1, NM_001282285.1 variant 2, NM_001282286.1 variant 3, and NR_104115.1 variant 4 and differ from each other either in the 5' UTR or in the 3' coding region. CpG site cg09418321 located in DYRK4 (Chr 12: 4569879, Build 36.1/hg 18) showed the highest decrease in methylation compared to the other CpG sites and healthy controls. A decreased methylation at the CpG sites in the promoter region could also be observed as already mentioned for the other genes in the accompanying CpG sites of DYRK4.

The Methylation Array also revealed an altered methylation status in FUT7 (NM_004479.3, fucosyltransferase 7) which showed a decreased DNA methylation in its promoter region. The most significant decrease in methylation state could be observed at the CpG site cp02679745 in FUT7 (Chr 9: 139047467, Build 36.1/hg 18). Nevertheless, the methylation level of all other flanking measureable CpG sites of FUT7 were significant lower in the 444 BC cases compared to healthy controls (n=444).

In a preferred embodiment of the present invention, the method for early diagnosing cancer or a susceptibility of developing cancer in a subject comprises determining genomic modifications and/or altered expression is determined of one or more genes selected from the group consisting of
- S 100P (NM_005980.2, S100 calcium binding protein P),
- SLC22A18 (NM_002555.5 variant 1, NM_183233.2 variant 2, Solute carrier family 22, member 18),
- DYRK4 (NM_003845.2 variant 1, NM_001282285.1 variant 2, NM_001282286.1 variant 3, and NR_104115.1 variant 4, dual-specificity tyrosine-(Y)-phosphorylation regulated kinase 4), and
- FUT7 (NM_004479.3, fucosyltransferase 7).

As mentioned above, in a preferred embodiment the method of the present invention comprises the determination of the methylation of at least one CpG site of any one of the mentioned genes, preferably of at least one CpG site as shown in Fig. 1, most preferably of at least the methylation of one or more CpG sites selected from the group consisting of CpG sites cg22266967 in S100P (Chr 4: 6746599, Build 36.1/hg18), cg21019522 in SLC22A18 (Chr 11: 2877365, Build 36.1/hg18), cg09418321 in DYRK4 (Chr 12: 4569879, Build 36.1/hg18), and cg02679745 in FUT7 (Chr 9:139047467, Build 36.1/hg18).

As shown in Table 8, the individual CpG sites of the four genes can differentiate cancer patients, in particular BC cases from controls with variant discriminatory power, but the significance of the methylation panel by taking all CpG sites into consideration is stronger than taking any one alone.

Therefore, in one embodiment the methylation status of at least 2, at least 3, at least 4, at least 5, at least 6, at least 7 of the CpG sites of the CpG sites located in the candidate genes is determined. In a preferred embodiment the methylation status of at least 2, at least 3, at least 4 of the CpG sites is determined.

As shown in Example 4 the methylation panel of each of the four candidate genes enabled a powerful discrimination of early stage BC cases from healthy controls, as shown by the area under curve (AUC) which was determined as 0.79; 95% CI, 0.75-0.83, and was efficient for younger women as well (age < 50, AUC = 0.84; 95% CI, 0.80-0.87) compared to other methods.

Therefore, in one embodiment, the subject to be analyzed in accordance with the method of the present invention is substantially healthy and does not show any symptoms or signs of cancer.

A great benefit of the method of the present invention is that the method enables the identification of cancer also in younger patients, *e.g.* younger woman suffering BC or being at risk thereof. For this reason, the method of the present invention may serve as an independent tool to detect BC compared to visual procedures, *i.e.* mammography. Accordingly, in one embodiment, the age of the subject to be analyzed in accordance with the method of the present invention is below the age commonly considered for cancer check-up by visual means or breast scanning, i.e. preferably under 50 years (< 50), more preferably below 45 (<45) or 40 years (<40).

Another advantage of the method described herein is that the panel of genes identified in accordance the present invention is indicative for subjects which are at risk of developing cancer because of having a hereditary handicap, for example inherited epigenetic changes as well with spontaneous developing cancer, *i.e.* genomic modifications and altered gene expression acquired during the lifetime of the subject. Accordingly, the subject to be analyzed in accordance with the method of the present invention may be a familiar cancer patient or a sporadic cancer patient.

The terms "biological sample", "sample", and "clinical sample" are used interchangeable herein and contains nucleic acids, in particular total, preferably genomic DNA, total RNA, preferably mRNA or proteins, preferably soluble proteins, or combinations thereof isolated from a subject and include but is not limited to, tissue biopsy, autopsy material, pathology specimens, sections of tissues, cells, body fluids, such as blood, sputum, serum, milk, saliva, or urine, or fractions and/or components thereof. As illustrated in the Examples blood samples have been used which provides the advantage of easy collection of the samples and the possibility to take recourse of blood samples collected previously for other diagnostic purposes. Therefore, in a preferred embodiment the sample from the subject to be analyzed in accordance with the method of the present invention is a body fluid, preferably peripheral blood or derived thereof; see also the Examples.

A "control" is to be understand as a sample or standard used for comparison with the experimental sample. The control may include a sample obtained from a healthy subject without cancer or a non-tumor sample. A "reference value" may be a standard reference value or range of values, *i.e.* such as determined from determined for the status of genomic modifications, *i.e.* including those which do not involve a change in the nucleotide sequence such as methylation and/or expression level of the candidate genes in a previously tested control samples. For example, the values determined in accordance with the present invention for the controls depicted in any one of the Tables below may generally serve as reference values. In this context, it is understood that for the purpose of the method of the present invention the term "control" also include embodiments where a sample from a subject previously determined to suffer from or be at risk of developing cancer may be the source of a control sample irrespective whether or not the subject has been diagnosed with a method of the present invention or an equivalent diagnostic tool. Similarly, the term "reference value" includes embodiments where the standard reference value or range of values has been determined to be indicative for the onset, presence or risk of developing cancer and thus may serve as a "positive" without the need for a control or reference value obtained from a healthy volunteer. Accordingly, in one embodiment, the values determined in accordance with the method of the present invention for subjects afflicted with cancer depicted in any one of the Tables below may generally serve as reference values.

Exemplary methods for the detection of the presence or absence and/or absolute or relative amount of methylation include those disclosed in Examples as well as, but not limited to a method selected from the group consisting of PCR, methylation-specific PCR, real-time methylation-specific PCR, PCR assay using a methylation DNA-specific binding protein, methylation-specific enzyme digestion, methylationspecific polymerase chain reaction (MSPCR), quantitative PCR, methylation-sensitive single nucleotide primer extension (MS-SnuPE), DNA chip-based assay, pyrosequencing, and bisulfite sequencing, and methylation profiling arrays, as well as methods known in the art. However, the methods may vary from each other, e.g. methylation profiling arrays may vary in structure, composition, and intended functionality, and may be based on either a macroarray or a microarray format, or a combination thereof. As described above, methylation can be measured by employing a methylation-specific enzyme digestion, which utilizes methylation sensitive restriction endonucleases for the differentiation between methylated and unmethylated cytosines. The assessment can then be performed utilizing Southem blot analysis or PCR. Methods for determining and analyzing methylation profiles are additionally disclosed in the international application WO2012/116019, the disclosures of which are incorporated herein by reference. In methods utilizing bisulfite treated DNA, such MSPCR, the genomic DNA is converted using bisulfite treatment. Bisulfite conversion can be perform utilizing methods known in the art as well as commercial kits, such as the EZ DNA Methylation Gold Kit (Zymo Research Corporation, Irvine, Califomia). Further methods are *e.g.* described in Krueger et al., Nature Methods 9, (2012), 145-15.

While the methylated cytosine in the 5'-CpG-3' region remains intact after the treatment, the unmethylated cytosine changes to uracil. Accordingly, based on the base sequence converted after bisulfite treatment, PCR primer sets corresponding to a region having the 5'-CpG-3' base sequence are constructed. The analysis of bisulfite treated DNA can be performed in different ways. On the one hand, the constructed primer pairs are two kinds of primer pairs, *i.e.* a primer pair which corresponds to the methylated base sequence (original sequence), and a primer pair which corresponds to the unmethylated base sequence (changed sequence). This allows the detection of genomic DNA, when it is converted with bisulfite and then amplified by PCR using the above two kinds of primer pairs. In particular, the PCR product is detected in the PCR mixture utilizing the primers corresponding to the methylated base sequence (original sequence), when the genomic DNA is methylated. In contrast, genomic DNA in the PCR mixture employing the primers corresponding to the unmethylated is detected, when the genomic DNA was unmethylated (changed sequence). This difference in methylation can then be quantitatively analyzed by agarose gel electrophoresis. On the other hand, only one pair of primers which can amplify both the methylated and unmethylation sequences may be designed. The methylation levels are then determined by the different mass between the adenine and cytosine vs. the guanine and cytosine. The mass analysis may be performed utilizing a MALDI-TOF analysis.

As shown in Examples 5 and 6, the method of the present invention could also be utilized for the diagnosis of other types of cancer, *e.g.* ovarian cancer (OvCa) and pancreatic cancer (PaCa).

In particular, the methylation level of 16 CpG sites in S100P, SLC22A18, and FUT7 showed a significantly lower methylation level in peripheral blood in sporadic OvCa cases than healthy controls. In addition, the potential clinical utility assessing the ROC curve analysis showed a robust discriminatory power for differentiating OvCa cases from healthy controls, see *e.g.* Table 11 and Fig. 4.

Additionally, also PaCa patients had a much lower methylation at all CpG sites of S100P compared to healthy controls. Therefore, in a preferred embodiment of the present invention, the subject wherein the cancer which is to be determined is affected with or at risk developing breast cancer (BC), ovarian cancer (OvCa), or pancreatic cancer (PaCa).

However, the method of the present invention cannot only be used for the general detection of cancer, but can also be utilized for the discrimination of the specific types of cancer, as shown in Table 11.

In particular, the methylation status of the genes SLC22A18 and FUT7 was shown to be more sensitive for the detection of OvCa than for BC. The CpG site SLC22A18_CpG_8 showed the strongest discriminatory power for the detection of OvCa (AUC = 0.82, Table 11), whereas it showed the lowest efficiency for the detection of BC (first validation round, AUC = 0.67; second validation round, AUC = 0.68, the lowest; third validation round, AUC = 0.60, the lowest; Table 8). In addition, the AUCs of the seven CpG sites of FUT7 ranged from 0.64 to 0.78 for the detection of OvCa (Table 11), whereas the AUCs for the detection of BC ranged from 0.58 to 0.67 (third validation round, Table 8). However, the most representative CpG site for the detection of OvCa and BC is identical, *i.e.* cg0267945 as shown in Table 8 and Table 11. Furthermore, determining the methylation status of the S100P revealed that the detection efficiency of S100P was similar for OvCa and BC, but with different representative loci, *i.e.* S100P_CpG_7 for OvCa and S100P_CpG_8 for BC, see *e.g.* Table 8 and Table 11.

In view of PaCa, S100P had a lower methylation status in all the five CpG sites of S100P than healthy controls, as shown in Table 12 and in particular CpG site S100P_CpG_7 represented an AUC of 0.93, see *e.g.* Table 13. In addition to the difference between subject affected with cancer and healthy controls, a larger methylation difference could be observed in males between PaCa cases and controls than in females in all the five detected CpG sites of S100P, see e.g. Table 12.

As mentioned above, and as set forth in the Examples, the four candidate genes show a different methylation status in the different types of cancer. Therefore, in one embodiment, the present invention relates to the method wherein the cancer can be discriminated by the methylation status in the different genes.

In a preferred embodiment of the present invention, the discrimination occurs due to the different methylation status in the specific CpG loci of the candidate genes, in particular those illustrated in the appended Examples, Tables and Figures. In this context, the method of the present invention does only allow the diagnosis of cancer at an early stage but also discriminating various stages of cancer, in particular if the methylation status of one or more specific CpG sites of S100P, SLC22A18, DYRK4, and FUT7 is determined.

Previously, a strong association between decreased methylation of HYAL2 (hyaluronoglucosaminidase 2) in peripheral blood and BC has been revealed; see European patent application EP 12 178 715.4 and subsequent applications thereof, the disclosure content of which incorporated herein by reference. Since in some embodiments of the present invention, for example the early detection of breast cancer including HYAL2 as an additional candidate gene may improve the significance of the diagnosis, in one embodiment the method of the present invention further comprises determining the status of methylation of at least one CpG site in the HYAL2 gene (NM_003773.4 variant 1, hyaluronoglucosaminidase 2), preferably located in the promoter region of the gene, most preferably at CpG site cg27091787.

As already mentioned above, the experiments performed in accordance with the present invention revealed that methylation of cytosine residues contained within CpG islands of certain genes has been inversely correlated with gene activity. As shown in Example 3 and Table 7 the correlation between the methylation status and gene expression was assessed. The gene expression analysis showed that the relative expression level of S100P, SLC22A18, and DKYR4 in leucocytes of sporadic BC cases was higher than in controls. Therefore, in one embodiment of the present invention the method for early diagnosing cancer or a susceptibility of developing cancer the expression level of one or more candidate genes is determined.

The expression level of the candidate genes may be determined by different means, for example on the RNA level or on the level of the expression product, i.e. determining the amount of mRNA and protein, respectively vis-à-vis a control or reference value.

The methods for providing a RNA sample includes but are not limited to general methods known in the art for RNA extraction. Additionally, RNA and specifically mRNA, can be isolated using any suitable technique known in the art, e.g. phenol-based extraction which is a common method for isolation of RNA. Phenol-based reagents contain a combination of denaturants and RNase inhibitors for cell and tissue disruption and subsequent separation of RNA from contaminants. Phenol-based isolation procedures are efficient methods for isolating large, small, and total RNA from biological samples that contain mRNA, miRNAs and siRNAs, and can recover RNA species in the 10-200- nucleotide range. These methods include but are not limited to extraction methods such as those using Guanidinium thiocyanate-phenol-chloroform such as TRIZOL™ or TRI REAGENT™. Additional methods include RNA silica-membrane purification of RNA, see *e.g.* in the Examples. General methods known in the art are disclosed in *e.g.* Ausubel et al., Current Protocols in Molecular Biology, John Wiley and Sons (1997) and Tan et al., J. Biomed. Biotechnol. 2009 (2009), 1-10.

Methods for the detection of gene expression include but are not limited to quantitative PCR, quantitative real time PCR (qPCR), reverse transcription (RT)-qPCR, microarray, *in situ* hybridization, *in situ* PCR, Serial Analysis of Gene Expression (SAGE), northern blotting, next-generation sequencing, Whole Transcriptome Shotgun Sequencing (WTSS), and Transcript Analysis and Affinity Capture (TRAC).

As already mentioned above, in one embodiment of the present invention, the method, wherein a RNA sample is provided from a subject and the expression level of one or more candidate genes is determined may be combined with the data obtained by the methylation analysis. In a preferred embodiment, the altered methylation and/or expression of the candidate genes is observed compared to a corresponding reference value or a control sample of a healthy subject which is indicative for the presence or risk and susceptibility of developing cancer. In a particular preferred embodiment the altered methylation and/or expression is a decreased methylation and/or over-expression, preferably in peripheral blood.

For genes encoding proteins, the expression level can be directly assessed by protein quantification utilizing methods known in the art. Therefore as an alternative or additional embodiment the protein expression can be determined. In particular, in one embodiment the method of the present invention comprises (a) providing a protein sample from a subject and (b) determining the protein expression level of one or more candidate proteins. The altered protein expression of the candidate proteins is compared to a corresponding reference value or a control sample of a healthy subject which is indicative for the presence or risk and susceptibility of developing cancer. The altered expression is preferably an over-expression of one or more of the candidate proteins, preferably in peripheral blood of BC, OvCa, and PaCa patients.

Methods for protein extraction and/or quantification are well known in the art include but are to those described in Ninfa *et al.,* Fundamental Laboratory Approaches for Biochemistry and Biotechnology, Chapter 4: Quantification of Protein Concentration, and Chapter 7: Overview of Protein Purification (2009).

One of the ways in which a protein can be detectably labeled is by linking the same to an antibody and using the linked product in an enzyme immunoassay (EIA) (Voller, A., "The Enzyme Linked Immunosorbent Assay (ELISA)" Microbiological Associates Quarterly Publication, Walkersville, Md., Diagnostic Horizons 2 (1978), 1-7); Voller et al., J. Clin. Pathol. 31 (1978), 507-520; Butler, Meth. Enzymol. 73 (1981), 482-523; Maggio, (ed.), Enzyme Immunoassay, CRC Press, Boca Raton, Fla., (1980); Ishikawa, et al., (eds.), Enzyme Immunoassay, Kgaku Shoin, Tokyo (1981). The protein, which is bound to the antibody, will react with an appropriate substrate, preferably a chromogenic substrate, in such a manner as to produce a chemical moiety which can be detected, for example, by spectrophotometric, fluorimetric or by visual means.

ELISA kits as well as antibodies for the candidate genes are commercially available, see *e.g.* ELISA kit #KA0093 for S100P from Abnova or anti-S100P antibody as well as antibodies against FUT7 are available from Abcam, Cambridge. Corresponding S100P antibodies has been also described in Parkkila *et al.,* BMC Clinical Pathology 8:2 (2008), 1-9. Solute carrier family 22 member 18 ELISA Kit #ABIN1158882 or a corresponding ELISA kit #ABIN1158882 from antibodies online. Anti-Dyrk4 antibodies are also commercially available at Santa Cruz Biotechnology, Heidelberg. However, corresponding methods for the production of antibodies are known to the person skilled in the art and are described, *e.g.,* in Harlow and Lane "Antibodies, A Laboratory Manual", CSH Press, Cold Spring Harbor (1988).

As already described above, the protein expression data may be combined with further methylation related biomarker detection methods, such as methylation analysis as well as non-methylation related biomarker detection, such as gene expression analysis methods to obtain a more precise cancer diagnosis and/or discrimination

Taking the above explanations into account, in one embodiment of the present invention the methylation data obtained may be combined with the non-methylation related biomarker detection methods, *e.g.* RNA expression analysis and/or protein expression analysis to obtain a more precise cancer diagnosis and/or discrimination of cancer and/or the type of cancer at an early stage or a verification of the results obtained by methylation analysis.

As used herein, the determination or measurement of the expression level of a gene or gene product, including mRNA or protein, refers to quantifying the amount of the gene or gene product present in a sample. Quantification can be either numerical or relative.

A decreased expression or downregulated expression refers to the expression of nucleic acid. It refers to any process which results in a decreased production of a gene product. In contrast, an increased expression or upregulated expression refers to any process which results in an increased production of a gene product. A gene product can be a primary transcript microRNA (pri-miRNA), precursor microRNA (pre-miRNA), mature microRNA or mRNA as well as a protein, amino acid, oligonucleotide.

In another embodiment of the present invention, the method comprises contacting the isolated genomic DNA with sodium bisulfite prior to the measurement of the methylation status of one or more methylated genomic CpG dinucleotide sequences of one or more of the candidate genes and thereby converting unmethylated cytosine residues to uracil in the genomic DNA, while methylated cytosine residues in the genomic DNA remains unchanged. Subsequently, the methylation status is measured of one or more methylated genomic CpG dinucleotide sequences. In a further embodiment, the method also includes the amplification of the sodium bisulfite treated genomic DNA. As shown in Fig. 1C the DNA methylation is determined using bisulfite-specific primers. In a preferred embodiment of the present invention the bisulfite-specific primers are selected from the group of primers amplifying the nucleotide sequence of S100P (NC_000004.12 Reference GRCh38 Primary Assembly, AC_000136.1 Alternate HuRef, NC_018915.2 Alternate CHM1_1.1), SLC22A18 (NC_000011.10 Reference GRCh38 Primary Assembly, NG_011512.1 RefSeqGene, NT_187585.1 Reference GRCh38 ALT_REF_LOCI_1, AC_000143.1 Alternate HuRef, NC_018922.2 Alternate CHM1_1.1), DYRK4 (NC_000012.12 Reference GRCh38 Primary Assembly, AC_000144.1 Alternate HuRef, NC_018923.2 Alternate CHM1_1.1), FUT 7 (NC_000009.12 Reference GRCh38 Primary Assembly, NG_007527.1 RefSeqGene, AC_000141.1 Alternate HuRef, NC_018920.2 Alternate CHM1_1.1) or fragments thereof or its bisulfite-converted sequence. Preferably, the primer or primer pair is selected from the group of primers comprising or consisting of the nucleotide sequence of SEQ ID NOs 5 and 6, 7 and 8, 9 and 10, and 11 and 12, respectively.

The present invention also relates to a kit comprising one or more containers filled with one or more ingredients of the above described method. For example the kit is suitable for the determination a methylation status and/or expression level in accordance with the present invention comprising one or more primers for amplifying a fragment comprising a CpG site and/or for analyzing the product amplified by such primer pair. In a preferred embodiment, the fragment comprising the CpG site is at least one fragment of one or more candidate genes. In a particular preferred embodiment the kit comprises a primer pair selected of one or more primers selected from the group pair amplifying the nucleotide sequence of S100P (NC_000004.12 Reference GRCh38 Primary Assembly, AC_000136.1 Alternate HuRef, NC_018915.2 Alternate CHM1_1.1), SLC22A18 (NC_000011.10 Reference GRCh38 Primary Assembly, NG_011512.1 RefSeqGene, NT_187585.1 Reference GRCh38 ALT_REF_LOCI_1, AC_000143.1 Alternate HuRef, NC_018922.2 Alternate CHM1_1.1), DYRK4 (NC_000012.12 Reference GRCh38 Primary Assembly, AC_000144.1 Alternate HuRef, NC_018923.2 Alternate CHM1_1.1), FUT 7 (NC_000009.12 Reference GRCh38 Primary Assembly, NG_007527.1 RefSeqGene, AC_000141.1 Alternate HuRef, NC_018920.2 Alternate CHM1_1.1) or fragments thereof or its bisulfite-converted sequence. Preferably, the primer or primer pair is selected from the group of primers comprising or consisting of the nucleotide sequence of SEQ ID NOs 5 and 6, 7 and 8, 9 and 10, and 11 and 12, respectively.

The kit utilized in accordance with the method of the present invention, will allow the early diagnosis of cancer or a susceptibility of developing cancer in a subject and/or the classification and/or discrimination of the cancer type. In a preferred embodiment, the methylation status and/or expression level including DNA, RNA, and protein, is determined using the above mentioned kit, wherein the altered methylation and/or expression level status compared to a corresponding reference value or control is indicative for the presence or risk and susceptibility of developing cancer. In a preferred embodiment, the altered methylation status and/or expression level is measured in peripheral blood. In a more preferred embodiment, the altered methylation status and/or expression level measured in peripheral blood is a decreased methylation status and/or over-expression. In addition or alternatively, the kit may further comprise instructions for carrying out said method and optionally standards for a control or reference as well as reagents and/or instructions for use in appropriate diagnostic assays. The results or output obtained by the kit can be provided in a paper output, a display on a screen, a graphical output, audible output. Furthermore, the data output includes but is not limited to numerical values, graphical presentations, quantitative informations, qualitative informations regarding the relative amount of methylation. Furthermore, information or guidelines for interpreting the data providing a cut-off value or methylation status and/or expression level that is indicative for the presence of cancer or the risk of developing cancer, *i.e.* the predisposition of the subject, can be included in the kit.

In an additional or as an alternative embodiment, the method of the present invention relates to a device for diagnosing cancer. This device comprises two units, *i. e.* an analyzing and an evaluation unit, which might be separated from each other as two independent units or devices or forming one unit. In a preferred embodiment the device is formed from one unit comprising the analyzing and evaluation unit. In one embodiment the analyzing unit comprises a detection agent for determining the methylation status of one or more candidate genes. In a further embodiment the analyzing unit determines the methylation level of one CpG site as described above. In addition, the analyzing unit can comprise a receptacle for a sample of a subject which is suspected to suffer from or being at risk of developing cancer, which might be e.g. a disposable test strip. The evaluation unit comprises a data processor having tangibly embedded an algorithm for carrying out a comparison of the amount determined by the analyzing unit with a stored reference and which is capable of generating an output file containing a diagnosis established based on the said comparison. The devices for diagnosing cancer in accordance with the methods described herein, allows the determination and/or discrimination of cancer and/or its classification at an early stage in a simple and effective manner.

As shown in the Examples and explanations herein, the determination and/or discrimination of cancer by determining the methylation and/or expression level is possible. Therefore, the present invention also relates to a method for monitoring the therapy of a subject being treated against cancer. In particular, the method comprises the steps of providing a sample from the subject and determining the expression level of the genes, CpG sites, and/or proteins, as described above. In a preferred embodiment, the sample utilized in the method for monitoring of therapy is or comprises DNA, RNA, and/or protein. In addition, the altered methylation status and/or expression profile compared to a reference sample, preferably DNA, RNA, and/or protein, obtained from the subject before or at an earlier stage of the treatment indicate the effectiveness of a treatment course undergoing the subject. In a preferred embodiment, the altered methylation status and/or expression profile is a decrease in methylation status and/or over-expression in peripheral blood of the above identified genes or proteins.

In addition, due to the possibility of the early deducing and discriminating or classifying cancer and its susceptibility a treatment regimen can be developed, in particular for the prophylactic treatment of subjects at risk to develop cancer, for example breast cancer due to family history. In this context one or more candidate genes can be utilized as biomarkers for the early detection of cancer as shown by the Examples and explanations above. Additionally, cancer treatment can be envisaged in view of the altered methylation status and/or expression level of the marker genes of the present invention. This is particular advantageous since an early treatment, *i. e.* before the onset of metastasis may be possible. A further advantage of an early stage therapy as well as perhaps a more precise treatment may be that possible side effects of cancer treatment, dependent on the severity of the cancer and treatment such as typical side effects of chemotherapy might be avoided.

In this context, in one embodiment the method of the present invention includes the provision an appropriate therapy for the subject affected or at risk developing cancer. In some embodiments, the therapy includes the administration of an anti-cancer agent for use in the treatment, prevention or amelioration of a cancer in a patient at an early stage that alters one or more of the candidate genes. In a preferred embodiment, the patient is characterized by a decreased methylation status of at least one CpG site of one or more candidate genes or an increased expression of one or more candidate genes as compared to a corresponding control sample of a healthy subject or reference value. Alternatively or additionally the patient has been diagnosed by the methods as described above. The patient who obtains a treatment with the anti-cancer agent may suffer from BC, OvCa, or PaCa.

As already mentioned above, an advantage of the present invention is that method enables the identification of cancer in younger patients. For this reason, the treatment with the anti-cancer agent for preventing, ameliorating, or treating cancer in a subject can occur at an early stage of cancer, where no signs or symptoms are visible. Therefore, the treatment might also serve as a kind of prevention to avoid the onset of cancer or symptoms thereof. Accordingly, in one embodiment, the age of the subject to be treated with an anti-cancer agent in accordance with the method of the present invention is below the age commonly considered for cancer check-up, *i.e.* preferably under 50 years (< 50), more preferably below 45 (<45) or 40 years (<40).

The anti-cancer agent can be administered to the subject at a suitable dose and can be effected by different ways, *e.g.* by intravenous, intraperitoneal, subcutaneous, intramuscular, intranasal, topical or intradermal administration or spinal or brain delivery, but also in aerosolic formulations and formulations for rectal or vaginal administration with a suitable carrier.

The dosage will be determined by the attending physician and clinical factors but can also be adjusted in accordance with the diagnosis as set forth above or the progression or regression of cancer which is to be assessed *e.g.* by the monitoring therapy of a subject being treated against cancer comprising the steps of (a) providing a sample from the subject being treated; and (b) determining the methylation status of at least one of the CpG sites as defined above or the expression level one or more candidate genes and/or proteins, wherein an increase of the methylation status and/or a decreased expression level compared to a reference sample obtained from the subject before or at an earlier stage of the treatment is indicative for the effectiveness of a treatment course undergoing by the subject of the therapy.

The anti-cancer agent can be one or more agent selected from the group consisting of, but not limited to small molecules such as Herceptin (Trastuzumab), Avastin (Bevacizumab), and the thyrosine kinase inhibitiors such as Lapatinib, Sorafinib oder Sunitini; Epirubicin, Doxorubicin, Cyclophosphamid, Taxol, Taxotere, Carboplatin, GnRH-analoga, estrogen receptor blocker such as Tamoxifen and Fulvestrant, aromatase inhibitors such as Arimidex, Femara, and Aromasin; Perjeta (Pertuzumab).

However, the anti-cancer agent can also be an agent which alters the epigenetics, e.g. the methylation status of genes. In this context, the agent may be a demethylating or methylating agent. In a preferred embodiment the agent is a methylating agent of the candidate genes.

The anti-cancer agent which is useful in the amelioration or the prevention of symptoms associated with cancer or the amelioration of general state of health of cancer patient can also be formulated in a pharmaceutical composition. The compositions of the present invention comprises an effective amount of the aforementioned anti-cancer agent. In addition or alternatively the composition may comprise a dosage regime for use of the anti-cancer agent in the treatment of the subject. In one embodiment, the composition of the present invention is a pharmaceutical composition and further comprises a pharmaceutically acceptable carrier. Pharmaceutical carriers are well known in the art and include but are not limited to phosphate buffered saline solutions, water, emulsions, such as oil/water emulsions, various types of wetting agents, and sterile solutions. The above mentioned compositions and/or pharmaceutical compositions comprising such carriers can be formulated by well-known conventional methods.

In a further embodiment, the present invention relates to a non-transient computer readable storage medium, comprising executable instructions for detecting cancer or a susceptibility of developing cancer, the executable instructions configured to:
(a) receive data characterizing the intensity of DNA methylation and/or expression level in one or more gene loci as defined in any one of claims 1 to 8;
(b) compare the intensity of DNA methylation and/or expression level to a model; and calculate a likelihood of cancer.

A non-transient computer readable storage medium useful and applicable in accordance with the present invention are well known in the art and described, for example in the international application WO2012/116019, the disclosure content of which incorporated herein by reference. In one embodiment the medium comprises executable instructions for detecting cancer or a susceptibility of developing said cancer, the executable instructions configured to receive data characterizing the intensity of the methylation status and/or expression level in one or more gene loci; and to compare the intensity of methylation or the level of protein or gene expression to a model; and calculate a likelihood of cancer. In particular, the cancer is discriminated by the methylation level in the specific CpG loci and/or the expression of the candidate genes. In a preferred embodiment of the present invention, the non-transient computer readable storage medium comprises any of the data illustrated in the appended Tables, Examples and Figures., for example for providing negative and/or positive reference values for performing the method of the present invention. In one embodiment, the non-transient computer readable storage medium is used in combination with the device of the present invention described hereinabove.

These and other embodiments are disclosed and encompassed by the description and examples of the present invention. Further literature concerning any one of the materials, methods, uses, and compounds to be employed in accordance with the present invention may be retrieved from public libraries and databases, using for example electronic devices. For example the public database "Medline" may be utilized, which is hosted by the National Center for Biotechnology Information (NCBI) and/or the National Library of Medicine at the National Institutes of Health (NLM.NIH). Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2. Several documents are cited throughout the text of this specification. Full bibliographic citations may be found at the end of the specification immediately preceding the claims. The contents of all cited references (including literature references, issued patents, published patent applications as cited throughout this application including the background section and manufacturer's specifications, instructions, etc.) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

Furthermore, the person skilled in the art will understand that any of the features and parameters such as ROC and AUC described in the examples and shown in the Tables and Figures generally relate to the embodiments characterized in the claims and described herein above. In addition, it is to be understood that each of those features and parameters may be used individually and isolated from the examples and/or in combination including any combination with the embodiments cited in the claims and in the preceding description.

### EXAMPLES

### Patients and methods

### Study population

The present study was approved by the Ethics Committee of the University of Heidelberg (Germany). All the cancer patients and healthy controls were Caucasian. All the recruited cases and controls gave written informed consent for the study. Genomic DNA was isolated from peripheral whole blood using DNA isolation kits from Qiagen. The leucocytes were immediately frozen in liquid nitrogen after isolation and stored at -80°C until use. DNA and RNA were isolated from leucocytes using AllPrep DNA/RNA/Protein Mini Kit from Qiagen. Detailed information for the samples was shown in Table 1. Please see the clinical data of the sporadic BC patients in Table 9.

### BC cases and matched controls,

Peripheral blood samples from 160 *BRCA1*/*2* mutation-negative index familial BC patients (first validation round) were collected by the centers of the German Consortium for Hereditary Breast and Ovarian Cancer in Heidelberg and Cologne. All the familial BC cases were recruited according to the criteria of family history described previously²⁶. Peripheral blood samples from 284 sporadic BC patients (189 in the second validation round and 95 in the third validation round) were collected at the time point of first BC diagnosis before any BC treatment and surgery at the University Hospital of Heidelberg. The clinical characteristics of sporadic BC patients were defined according to the American Joint Committee on Cancer (AJCC) cancer staging manual²⁷. Peripheral blood samples from 349 healthy female controls (160 in the first validation round and 189 in the second validation round) were collected from blood donors by the German Red Cross Blood Service of Baden-Württemberg-Hessen. Peripheral blood samples from 95 healthy female controls (third validation round) were collected at the University Hospital of Heidelberg. All the cases and controls in the third validation round were processed with the same manner in parallel.

Leucocytes were isolated from peripheral blood using red blood cell lysis buffer as described previously²⁸ within four hours after blood collection at the University Hospital of Heidelberg. All the leucocytes from cases and controls were processed in parallel.

### OvCa cases and matched controls,

Peripheral blood samples from 84 sporadic OvCa patients and 148 healthy female controls were collected at the University Hospital of Heidelberg. All the cases and controls were processed with the same manner in parallel.

### PaCa cases and matched controls,

Peripheral blood samples from 147 sporadic PaCa patients (80 male cases and 67 female cases) were collected at the University Hospital of Heidelberg. The PaCa cases were specially selected with higher percentage of early stage cases. Peripheral blood samples from 191 healthy female controls (115 male cases and 76 female cases) were collected from blood donors by the German Red Cross Blood Service of Baden-Württemberg-Hessen.

### DNA methylation assays

Genome-wide methylation screening was performed using the Illumina Infinium Methylation27 Bead Array according to the manufacturer's recommendations²⁹. All the 96 samples passed the quality control.

MALDI-TOF mass spectrometry (Sequenom) described by Breitling *et al.*^{14,15} was used in all the validation and further exploring rounds. The bisulfite-specific primers (no SNPs in the primers) and PCR amplicons are presented in Supplementary Figure 1. All the cases and controls were processed in parallel.

### Quantitative Real-time PCR for RNA Expression

RNA was transcribed into cDNA using TaqMan® Reverse Transcription Reagents (Applied Biosystems). Quantitative real-time PCR was performed using a LightCycler480 (Roche) in combination with TaqMan gene expression assays (Applied Biosystems) for S100P, SLC22A18, DYRK4 gene and housekeeping gene HPRT1 as endogenous control. Relative expression of genes for each sample was calculated according to the ΔΔCt method by normalization to HPRT1. All the cases and controls were processed in parallel.

### Statistical Analysis

The Illumina 27K Array data were processed by the Illumina BeadStudio software with default settings. Probes with detection P-value > 0.01 were removed and samples were quantile-normalized. Association of probes with case/control status was assessed by beta-regression models with a logistic link and associated Wald tests using the R package betareg v2.2-3³⁰. Likelihood ratio tests were used to compare the case/control model with the nested model for chip differences in order to identify possible false hits due to confounding by chip effects. Multiple testing adjustments were done with the Benjamini-Hochberg method controlling the false discovery rate at the level of 0.05. All analysis was performed with the statistical software R v2.11.1.

All the statistical analyses of MassARRAY and gene expression data were conducted by SPSS Statistics 17.0 software. The correlations were assessed by Spearman's rank correlation coefficients. Logistic regression models and non-parametric tests were used for comparisons between two and multiple groups. The results of logistic regression were adjusted for possible confounding effects of age and different measurement batches by including additional co-variables in the logistic regression models. Receiver operating characteristic (ROC) curve analysis was performed to assess the discriminatory power of methylation levels.

### Example 1: The identification of BC-associated differential methylation in S100P, SLC22A18, DYRK4 and FUT7 in peripheral blood by Methylation Array

A genome-wide methylation screen was performed using Human Methylation27 BeadChip (Illumina) in 72 familial BC cases and 24 healthy controls (the discovery round in Table 1). Besides the most significant CpG site cg27091787 in the hyaluronoglucosaminidase 2 gene (*HYAL2*) which has been validated in a previous study, the other significant CpG sites need further validation as well. 12 CpG sites with methylation intensity difference between cases and controls larger than 5% and with Bonferroni-adjusted *p*-value less than 0.05 (adjustedp-value < 1.0 × 10⁻⁶) were selected for the amplicon design and optimization (Figure 1A). In each amplicon, the Illumina CpG should be covered and be measurable by Sequenom MALDI-TOF (matrix-assisted laser desorption ionization time-of-flight) mass spectrometry. The PCR amplicons may cover multiple flanking CpG sites. As a result, only seven CpG sites can be successfully amplified by PCR and proceed for verification by Sequenom MALDI-TOF mass spectrometry.

### Example 2: The validation of BC-associated differential methylation in S100P, SLC22A18, DYRK4 and FUT7 in peripheral blood in three independent rounds

To verify the seven differential methylation sites in peripheral blood DNA between familial BC cases and controls, an independent sample set was investigated by MALDI-TOF mass spectrometry (160 familial BC cases and 160 matched healthy controls, the first validation round shown in Table 1). Three CpG sites can not been verified in the first validation round. The other four CpG sites, cg22266967 in S100P, cg21019522 in SLC22A18, cg09418321 in DYRK4 and cg02679745 in FUT7, which showed BC-associated decreased methylation in the discovery round (Table 2) also showed significantly lower methylation in familial BC cases than control in the first validation round (p < 4.0 × 10⁻⁶ for all four CpG sites by logistic regression, Table 3). Moreover, the methylation levels of all other 15 flanking measurable CpG sits of the four genes were significantly lower in the 160 familial BC cases than in 160 healthy controls (p < 0.001 for all CpG sites by logistic regression, first validation round in Table 3). An inter-quartile analysis^{14,31} was then carried out to evaluate the association between the methylation levels of CpG sites and the risk of familial BC. Compared to the quartile with the highest methylation level (Q4), the three lower quartiles of methylation levels (Q1-Q3) in all 19 CpG sites were associated with up to 20.5-fold increased risk of familial BC (Table 4). When taken all the 19 CpG sites into consideration as a methylation panel, an index can be calculated by logistic regression. The methylation panel was strongly associated with familial BC (p = 1.58 × 10⁻¹⁷ by logistic regression, first validation round in Table 3) and the lowest quartile of the panel index was associated with 30.5-fold of increased risk of familial BC compared to the highest quartile (Q1 vs. Q4, OR = 30.52, 95% CI 12.47-74.73, p = 7.25 × 10⁻¹⁴, Table 4).

Next, the differential methylation in S100P, SLC22A18, DYRK4 and FUT7 was validated in peripheral blood DNA between sporadic BC cases and healthy controls. To exclude the effects of treatment, only sporadic BC samples obtained at the time of BC first diagnosis before any BC treatment or surgery were used. In the second validation round (189 sporadic BC cases and 189 matched healthy controls, Table 1), except S100P_CpG_4, all the other 18 CpG sites had significantly lower methylation levels in the sporadic BC cases than healthy controls (all p < 0.005 by logistic regression, Table 2) and were associated with up to 12.9-fold increased risk of sporadic BC in their lower quartiles of methylation levels (Q1-Q3) compared to the highest quartile (Q4) (Table 5). The methylation panel of all 19 CpG sites was strongly associated with sporadic BC (p = 4.50 × 10⁻¹⁸ by logistic regression, second validation round in Table 3) and the lowest quartile of the panel index was associated with 26.5-fold of increased risk of sporadic BC compared to the highest quartile (Q1 vs. Q4, OR = 26.52, 95% CI 11.61-60.56, *p* = 7.27 × 10⁻¹⁵, Table 5).

In the third validation round, the investigation was performed on 95 sporadic BC cases and 95 matched healthy controls which were all collected at the University hospital of Heidelberg and were all processed with the same manner in parallel (Table 1). Although the sample size of the third validation round is limited, 15 CpG sites showed significantly lower methylation levels in the sporadic BC cases than healthy controls (all *p* < 0.005 by logistic regression, third validation round in Table 2) and were associated with up to 20.1-fold increased risk of sporadic BC in their lower quartiles of methylation levels (Q1-Q3) compared to the highest quartile (Q4) (Table 6). The methylation panel of all 19 CpG sites indicated a robust association with sporadic BC (p = 3.05 × 10⁻¹³ by logistic regression, third validation round in Table 3) and the lowest quartile of the panel index was associated with 68.1-fold of increased risk of sporadic BC compared to the highest quartile (Q1 vs. Q4, OR = 68.08, 95% CI 16.84-275.21, *p* = 3.17 × 10⁻⁹, Table 6).

### Example 3: The correlation between the methylation and expression of S100P, SLC22A18 and DYRK4 in leucocytes

To understand the correlation between the DNA methylation and gene expression, peripheral blood leucocytes was collected from 36 sporadic BC patients and 36 healthy controls at the University Hospital of Heidelberg (Table 1). Since the expression assay of FUT7 did not work efficiently, only the methylation and expression of S100P, SLC22A18 and DYRK4 were determined. Except S100P_CpG_4, all CpG sites in the peripheral blood DNA from sporadic BC cases showed significantly lower methylation than the methylation levels in healthy controls (Table 7). The relative expression level of S100P in the leucocytes of sporadic BC cases was almost three times higher than in controls (3.47 (inter quartile range (IQR) = 1.23-20.77) and 1.31 (IQR = 0.72-2.07) for sporadic BC cases and controls respectively, *p* = 0.001 by Mann-Whitney U test, Table 7). The relative expression level of SLC22A18 and DKYR4 in the leucocytes of sporadic BC cases was slightly higher than controls (Table 7). Except S100P_CpG_4, the methylation levels of all the 9 CpG sites in the S100P and SLC22A18 were strongly and inversely correlated with the expression of S100P and SLC22A18 respectively with all Spearman rho < - 0.5 (p < 1.0 × 10⁻⁵ for all, Table 7). The methylation levels two CpG loci in DYRK4 were not correlated with the expression of DYRK4 (for all, Spearman rho > - 0.1, *p* > 0.5, Table 7).

### Example 4: The clinical application of the methylation panel of S100P, SLC22A18, DYRK4 and FUT7 as a marker for the early detection of BC

To estimate the potential clinical utility of the methylation panel of S100P, SLC22A18, DYRK4 and FUT7 as a marker for the early detection of BC, receiver operating characteristic (ROC) curve analyses were performed. The methylation panel showed robust discriminatory power for differentiating BC cases from healthy controls in all the three validation rounds (first validation round, area under curve (AUC) = 0.82, 95% CI: 0.77-0.87; second validation round, AUC = 0.79, 95% CI: 0.74-0.84; third validation round, AUC = 0.86, 95% CI: 0.81-0.91, Figure 2 and Table 8). It was further investigated if the age has influence on the methylation panel for the breast cancer detection. An outstanding discriminatory power of the methylation panel for breast cancer detection was observed in the younger women (age < 50 years: 209 BC cases vs. 221 controls, AUC = 0.84, 95% CI: 0.80-0.88, sensitivity = 82% when specificity = 70%, Figure 3A and Table 8), whereas the detection power for the older women was sufficient as well (age ≥ 50 years: 232 BC cases vs. 223 controls, AUC = 0.76, 95% CI: 0.72-0.81, Figure 3B and Table 8). Moreover, the methylation panel of the four genes could detect breast cancer at very early stage (127 sporadic BC cases of stages 0&I vs. 444 controls, AUC = 0.79, 95% CI: 0.75-0.83, sensitivity = 74% when specificity = 70%, Figure 3C and Table 8) and before the micro-metastasis in lymph nodes (190 sporadic BC cases no involved lymph node vs. 444 controls, AUC = 0.80, 95% CI: 0.76-0.83, sensitivity = 75% when specificity = 70%, Figure 3D and Table 8). The individual CpG sites of in four genes can also differentiate BC cases from controls with variant discriminatory power, but all were weaker than the methylation panel by taking all CpG sites into consideration (Table 8)

To interrogate if the methylation levels of S100P, SLC22A18, DYRK4 and FUT7 is sensitive to breast cancer in general or only to certain specific breast cancer subgroups, the relationship between the methylation levels of all 19 CpG sites in the four genes and the characteristics of the 284 sporadic BC cases was further analyzed. The methylation levels of S100P, SLC22A18, DYRK4 and FUT7 showed no correlation with tumor stage, tumor size, status for lymphnode involvement, grading, and the status of ER, PR and Her2 receptors (Table 9). All the CpG sites in SLC22A18 and most of the CpG sites in S100P showed significantly lower methylation in the premenopausal women (Table 9), which might due to the association of these CpG loci and age (data not shown). In together, the methylation panel of four genes (S100P, SLC22A18, DYRK4 and FUT7) has a similar and robust discriminatory power for the detection of variant subgroups of breast cancer, representing a promising prescreening blood-based marker for BC early detection.

### Example 5: Blood-based methylation of S100P, SLC22A18, FUT7 and DYRK4 as a marker for the early detection of OvCa

Due to certain shared characteristics, markers identified in one type of cancer are commonly proved to be efficient for another type of cancer. Hereby the methylation level of S100P, SLC22A18 and FUT7 was investigated in peripheral blood of 84 sporadic OvCa patients compared to 148 healthy controls (Table 1). The methylation levels of 16 CpG sites in the three genes (except S100P_CpG_4) showed significantly lower methylation levels in the peripheral blood from sporadic OvCa cases than healthy controls (all *p* < 0.01 by logistic regression, Table 10). The methylation panel of all 17 CpG sites was strongly associated with sporadic BC (*p* = 1.38 × 10⁻¹⁵ by logistic regression, Table 10).

Further, the potential clinical utility of the methylation panel of the three genes (S100P, SLC22A18 and FUT7) was estimated as a marker for the early detection of OvCa by ROC curve analyses. The methylation panel of the three genes showed robust discriminatory power for differentiating OvCa cases from healthy controls (AUC = 0.89, 95% CI: 0.84-0.94, Figure 4 and Table 11). Notably, the methylation levels of SLC22A18 and FUT7 were more sensitive for the detection of OvCa than BC. The AUCs of the four CpG sites of SLC22A18, ranged from 0.77 to 0.82 for the detection of OvCa (Table 11), whereas the AUCs for the detection of BC ranged from 0.60 to 0.67 (third validation round, all the BC cases and control from the university hospital of Heidelberg, which is the same as the recourses of the OvCa cases and controls, Table 8). Interestingly, SLC22A18_CpG_8 showed the strongest discriminatory power for the detection of OvCa (AUC = 0.82, Table 11), whereas SLC22A18_CpG_8 had the lowest efficiency for the detection of BC (first validation round, AUC = 0.67; second validation round, AUC = 0.68, the lowest; third validation round, AUC = 0.60, the lowest; Table 8). Similarly, the AUCs of the seven CpG sites of FUT7 range from 0.64 to 0.78 for the detection ofOvCa (Table 11), whereas the AUCs for the detection of BC was from 0.58 to 0.67 (third validation round, Table 8). The most representative CpG site in FUT7 for the detection of OvCa and BC is the same one, cg0267945 (Table 8 and Table 11). The detection efficiency of S100P was similar for OvCa and BC, but with different most representative loci (S100P_CpG_7 for OvCa and S100P_CpG_8 for BC, Table 8 and Table 11).

The detection of OvCa with the four gene methylation panel will be even improved when the methylation level of DYRK4 in OvCa is determined.

### Example 6: Blood-based methylation of S100P, SLC22A18, FUT7 and DYRK4 as an outstanding marker for the early detection of PaCa

Next, the blood-based methylaiton level of S100P was analyzed in 147 sporadic PaCa patients compared to 191 healthy controls (Table 1). The investigations for the PaCa-associated methylation in SLC22A18, FUT7 and DYRK4 are in process. The peripheral blood of PaCa patients had much lower methylation in all the five CpG sites of S100P than healthy controls (all *p* < 1.0 × 10⁻¹⁸ by logistic regression, Table 12), especially at CpG site S100P_CpG_7, which the median of methylation in PaCa cases in just half of the controls (median of methylation in PaCa cases = 0.32, median of methylation in controls = 0.64, *p* = 5.5 × 10⁻²⁰ by logistic regression, Table 12). The samples were further stratified by gender and noticed that the males had larger methylation difference between PaCa cases and controls than the female in all the five detected CpG sites of S100P (Table 12). The gender related methylation difference was because the male controls had significantly higher S100P methylation than the female controls (p < 0.05 for all CpG sites of S100P), but not because the methylation difference in the male and female PaCa patients.

The methylation levels of the CpG sites in S100P showed extraordinary efficiency for the early detection of PaCa (AUC from 0.73 to 0.93), particularly, the S100P_CpG_7 represented an AUC of 0.93 (Table 13). Taken all the CpG sites in S100P together, the methylation level of S100P panel could distinguish PaCa cases from controls with an AUC of 0.95, 95% CI: 0.93-0.97, the sensitivity is 94% when the specificity is 83% (Figure 5A and Table 13). The relationship between the methylation levels of the five S100P CpG sites the clinical characteristics of the PaCa cases was further analyzed. The methylation levels of S100P showed no correlation with tumor stage, tumor size, status of lymphnode involvement, status of metastasis and grading (Table 14). Thus, the methylation levels of S100P have a similar and outstanding discriminatory power for the detection PaCa with variant clinical status, representing a promising blood-based marker with extraordinary power for the early detection of PaCa (Stage 0&I&II PaCa vs. controls, AUC = 0.94, 95% CI: 0.90-0.97, Figure 5B and Table 13). Next, the relationship between gender and S100P methylation was examined in peripheral blood. Although the PaCa patients showed no gender related S100P methylation difference (p > 0.05 for all five CpG sites), the male healthy people had higher S100P methylation in the blood than the female healthy people (*p* < 0.02 for all five CpG sites). Thus, the methylation level of S100P had higher detection efficiency for the male PaCa patients than the females. For the detection of PaCa in male patients, the blood-based methylation of S100P panel presented an outstanding AUC of 0.98 (sensitivity = 1.00, specificity = 0.86, Figure 5C and Table 13). The discriminatory power of S100P_CpG_7 is also robust for the PaCa in female patients (AUC = 0.92, sensitivity = 0.83, specificity = 0.90, Figure 5D and Table 13).

The detection of PaCa with the four gene methylation panel will be even improved when the methylation levels of FUT7, SLC22A18 and DYRK4 in PaCa are determined.

In summary, the methylation panel of S100P, SLC22A18, and FUT7 genes enables a powerful discrimination of OvCa cases from healthy controls (AUC = 0.89; 95% CI, 0.84-0.94). It would also be observed that the blood-based methylation level of SLC22A18 and FUT7 is more efficient for the detection of OvCa than BC. Impressively, the blood-based methylation level of S100P represented an outstanding detection effect for the PaCa (AUC = 0.95; 95% CI, 0.93-0.97), extraordinary power for the early detection of PaCa (Stage 0&I&II PaCa vs. controls, AUC = 0.94, 95% CI: 0.90-0.97), and particularly sensitive to male PaCa patients (AUC = 0.98; 95% CI, 0.97-1.00).

Interestingly, the CpG sites of these genes showed altered sensitivity to different cancers. The most representative CpG sites of one gene for the detection of one cancer may be the least sensitive CpG sites for the detection of other cancers (e.g., SLC22A18_CpG_4 and SLC22A18_CpG_8, S100P_CpG_7, Table 15). Thus, the determination of methylation level in specific loci provides a marker for the specific cancer.

Therefore, with the characteristics of non-invasive sampling and quantitative analysis, the utilization of the methylation and expression panel of four genes (S100P, SLC22A18, DYRK4 and FUT7) in peripheral blood facilitates the early detection of BC, OvCa and PaCa and the development of therapeutic strategy for preventing cancer and early treatment, respectively.

### Example 7: Blood-based methylation of S100P, SLC22A18, FUT7 and DYRK4 as an outstanding marker for the early detection of BC, OvCa, and/or PaCa

To further validate the results obtained by the Illumina Infinium Methylation 27 Bead Array (Illumina 27K Array) and MassARRAY, peripheral blood DNA was obtained from 48 sporadic breast cancer cases and 48 matched healthy controls and genome-wide methylation screening was performed using the Illumina Infinium Methylation 450 Bead Array according to the manufacturer's recommendations. The Illumina 450K Array data were processed by the Illumina BeadStudio software with default settings. The statistical analyses have normalized the color bias of the array, and have adjusted the variants like group, batch and age.

The 450K array had analysed 10 CpG sites in S100P gene. In Table 16, 4 out of the 10 investigated CpG sites in S100P showed BC-associated significant difference (pvalWald_Group < 0.05, meandiff > 0.15). All the four CpG sites are located from the promoter region (200 bp upstream of the transcript start site) till the first exon of S100P, covering a region of about 300-400 bp (chr4:6746220 - chr4: 6746599, build36.1/hg18). The CpG site cg22266967 (chr4: 6746599), which showed BC-associated significant difference by MassARRAY, was validated again by the Illumina 450K (pvalWald_Group = 0.0014, Table 1). Except cg22266967, the other CpG sites analysed by the MassARRAY were not included in the Illumina 450K Array.

The 450K array had analysed 91 CpG sites in SLC22A18 gene. In Table 17, 22 out of the 91 investigated CpG sites in SLC22A18 showed BC-associated significant difference (pvalWald_Group < 0.05, meandiff > 0.01). The 450K array data showed a 700-800 bp BC-associated differential methylation region in SLC22A18 (chr11: 2920189 - chr11: 2920819, build36.1/hg18), which is located at the promoter region or gene body region (transcript variants), and contains 12 differentially methylated CpG sites (Table 2). All the CpG sites which showed BC-associated significant difference by MassARRAY (cg21019522, SLC22A18_CpG_4/cg22315192, SLC22A18_CpG_6/ cg25427871 and SLC22A18_CpG_8/cg05752118) were validated by the Illumina 450K (pvalWald_Group < 0.05, Table 17).

The 450K array had analysed 8 CpG sites in DYRK4 gene. In Table 18, 2 out of the 8 investigated CpG sites in DYRK4 showed BC-associated significant difference (pvalWald_Group < 0.05, meandiff > 0.01). The two BC-associated CpG sites by Illumina 450K were all located at the promoter region (200 bp upstream of the TSS) and the 5'UTR. The CpG site cg09418321, which showed BC-associated significant difference by MassARRAY, was validated by the Illumina 450K (pvalWald_Group = 0.0011, Table 18. The other CpG site (DYRK4_CpG_3) analysed by the MassARRAY was not included in the Illumina 450K Array.

The 450K array had also analysed 6 CpG sites in FUT7 gene. In Table 19, 4 out of the 6 investigated CpG sites in FUT7 showed BC-associated significant difference (pvalWald_Group < 0.05, meandiff > 0.01). Three BC-associated CpG sites are all located at the promoter region and the 5'UTR (chr9: 139926740 - chr9: 139927646, build36.1/hg18). One CpG site (cg02971262) is located at the gene body (chr9: 139925395). The CpG site cg02679745, which showed BC-associated significant difference by MassARRAY, was validated by the Illumina 450K (pvalWald_Group = 0.00003, Table 19). The other CpG site analysed by the MassARRAY was not included in the Illumina 450K Array.

### References

1. World CanCer report 2008, International Agency for Research on Cancer, 2008
2. Benson JR, Jatoi I, Keisch M, et al: Early breast cancer. Lancet 373:1463-79, 2009
3. Jemal A, Bray F, Center MM, et al: Global cancer statistics. CA Cancer J Clin 61:69-90, 2011
4. Punglia RS, Morrow M, Winer EP, et al: Local therapy and survival in breast cancer. N Engl J Med 356:2399-405, 2007
5. Clarke-Pearson DL: Clinical practice. Screening for ovarian cancer. N Engl J Med 361:170-7, 2009
6. Kobayashi E, Ueda Y, Matsuzaki S, et al: Biomarkers for screening, diagnosis, and monitoring of ovarian cancer. Cancer Epidemiol Biomarkers Prev 21:1902-12, 2012
7. Goonewardene TI, Hall MR, Rustin GJ: Management of asymptomatic patients on follow-up for ovarian cancer with rising CA-125 concentrations. Lancet Oncol 8:813-21,2007
8. Tempero MA, Klimstra D, Berlin J, et al: Changing the way we do business: recommendations to accelerate biomarker development in pancreatic cancer. Clin Cancer Res 19:538-40, 2013
9. Hidalgo M: Pancreatic cancer. N Engl J Med 362:1605-17, 2010
10. Yadav D, Lowenfels AB: The epidemiology of pancreatitis and pancreatic cancer. Gastroenterology 144:1252-61, 2013
11. Esteller M: Epigenetics in cancer. N Engl J Med 358:1148-59, 2008
12. Heyn H, Esteller M: DNA methylation profiling in the clinic: applications and challenges. Nat Rev Genet 13:679-92, 2012
13. Laird PW: The power and the promise of DNA methylation markers. Nat Rev Cancer 3:253-66, 2003
14. Breitling LP, Salzmann K, Rothenbacher D, et al: Smoking, F2RL3 methylation, and prognosis in stable coronary heart disease. Eur Heart J, 2012
15. Breitling LP, Yang R, Korn B, et al: Tobacco-smoking-related differential DNA methylation: 27K discovery and replication. Am J Hum Genet 88:450-7, 2011
16. Ito Y, Koessler T, Ibrahim AE, et al: Somatically acquired hypomethylation of IGF2 in breast and colorectal cancer. Hum Mol Genet 17:2633-43, 2008
17. Potapova A, Hoffman AM, Godwin AK, et al: Promoter hypermethylation of the PALB2 susceptibility gene in inherited and sporadic breast and ovarian cancer. Cancer Res 68:998-1002, 2008
18. Radpour R, Kohler C, Haghighi MM, et al: Methylation profiles of 22 candidate genes in breast cancer using high-throughput MALDI-TOF mass array. Oncogene 28:2969-78,2009
19. Widschwendter M, Siegmund KD, Muller HM, et al: Association of breast cancer DNA methylation profiles with hormone receptor status and response to tamoxifen. Cancer Res 64:3807-13, 2004
20. Iwamoto T, Yamamoto N, Taguchi T, et al: BRCA1 promoter methylation in peripheral blood cells is associated with increased risk of breast cancer with BRCA1 promoter methylation. Breast Cancer Res Treat 129:69-77, 2011
21. Widschwendter M, Apostolidou S, Raum E, et al: Epigenotyping in peripheral blood cell DNA and breast cancer risk: a proof of principle study. PLoS One 3:e2656, 2008
22. Flanagan JM, Munoz-Alegre M, Henderson S, et al: Gene-body hypermethylation of ATM in peripheral blood DNA of bilateral breast cancer patients. Hum Mol Genet 18:1332-42, 2009
23. Brennan K, Garcia-Closas M, Orr N, et al: Intragenic ATM Methylation in Peripheral Blood DNA as a Biomarker of Breast Cancer Risk. Cancer Res 72:2304-2313, 2012
24. Koestler DC, Marsit CJ, Christensen BC, et al: Peripheral blood immune cell methylation profiles are associated with nonhematopoietic cancers. Cancer Epidemiol Biomarkers Prev 21:1293-302, 2012
25. Marsit CJ, Koestler DC, Christensen BC, et al: DNA methylation array analysis identifies profiles of blood-derived DNA methylation associated with bladder cancer. J Clin Oncol 29:1133-9, 2011
26. Meindl A: Comprehensive analysis of 989 patients with breast or ovarian cancer provides BRCA1 and BRCA2 mutation profiles and frequencies for the German population. Int J Cancer 97:472-80, 2002
27. Hayes DF, Allred C, Anderson BO, et al: Breast cancer in Edge SB, Byrd DR, Compton CC, et al (eds): AJCC cancer staging manual (ed seventh edition). New York, Springer, 2010, pp 345-376
28. Alan H L, Andrew R C, Davor B, et al: A method for ameliorating autoimmune disease by passive transfer of IVIg-primed leukocytes. Nature Protocol, 2006
29. Steemers FJ, Chang W, Lee G, et al: Whole-genome genotyping with the single-base extension assay. Nat Methods 3:31-3, 2006
30. Simas AB, Barreto-Souza W, Rocha AV: Improved Estimators for a General Class of Beta Regression Models. Computational Statistics and Data Analysis 54(2):348-366, 2010
31. Moore LE, Pfeiffer RM, Poscablo C, et al: Genomic DNA hypomethylation as a biomarker for bladder cancer susceptibility in the Spanish Bladder Cancer Study: a case-control study. Lancet Oncol 9:359-66, 2008
32. Ludwig JA, Weinstein JN: Biomarkers in cancer staging, prognosis and treatment selection. Nat Rev Cancer 5:845-56, 2005
33. Bast RC, Jr., Ravdin P, Hayes DF, et al: 2000 update of recommendations for the use of tumor markers in breast and colorectal cancer: clinical practice guidelines of the American Society of Clinical Oncology. J Clin Oncol 19:1865-78, 2001
34. Bianchini F, Vainio H: IARC Handbooks of Cancer Prevention: Breast Cancer Screening. Lyon, Lyon IARC Press, 2002
35. Issa JP: DNA methylation as a clinical marker in oncology. J Clin Oncol 30:2566-8, 2012

**Table 1. Sample description**

| **BC cases and matched controls** | | | | | | | | | | | |
|---|---|---|---|---|---|---|---|---|---|---|---|
| **Rounds** | **Sample types** | **Assays** | **Groups** | **Sample resourses** | **Target N** | **Mean of age (range)** | **Median of age** | **Assayed N (call rate %)** | | | |
| | | | | | | | | ***S100P*** | ***SLC22A18*** | ***DYRK4*** | ***FUT7*** |
| Discovery round | Peripheral blood DNA | Illumina 27K assay | controls | Blood donor from Mannheim | 24 | 48.5 (32-66) | 44.5 | 24 (100.0%) | 24 (100.0%) | 24 (100.0%) | 24 (100.0%) |
| | | | familial BC cases | Hereditary breast cancer centers in Heidelberg and Cologne | 72 | 47.0 (26-78) | 46 | 72 (100.0%) | 72 (100.0%) | 72 (100.0%) | 72 (100.0%) |
| First validation round | Peripheral blood DNA | MassARRAY | controls | Blood donor from Mannheim | 160 | 45.6 (30-67) | 44 | 160 (100.0%) | 156 (97.5%) | 18 (98.8%) | 156 (97.5%) |
| | | | familial BC cases | Hereditary breast cancer centers in Heidelberg and Cologne | 160 | 46.7 (24-78) | 46 | 159 (99.4%) | 18 (98.8%) | 159 (99.4%) | 18 (98.8%) |
| Second validation round | Peripheral blood DNA | MassARRAY | controls | Blood donor from Mannheim | 189 | 61.2 (31-69) | 64 | 189 (100.0%) | 188 (99.5%) | 189 (100.0%) | 189 (100.0%) |
| | | | sporadic BC cases | University hospital of Heidelberg | 189 | 59.6 (32-87) | 60 | 188 (99.5%) | 188 (99.5%) | 188 (99.5%) | 187 (98.9%) |
| Third validation round | Peripheral blood DNA | MassARRAY | controls | University hospital of Heidelberg | 95 | 41.5 (24-63) | 43 | 95 (100.0%) | 94 (98.9%) | 94 (98.9%) | 95 (100.0%) |
| | | | sporadic BC cases | University hospital of Heidelberg | 95 | 48.4 (29-61) | 48 | 94 (98.9%) | 95 (100.0%) | 95 (100.0%) | 95 (100.0%) |
| *HYAL2* methylation and expression in leucocytes | DNA and RNA from peripheral blood leucocytes | MassARRAY, real-time PCR | controls | University hospital of Heidelberg | 36 | 38.8 (21-61) | 40 | 36 (100.0%) | 36 (100.0%) | 35 (97.2%) | - |
| | | | sporadic BC cases | University hospital of Heidelberg | 36 | 58.1 (29-81) | 57 | 35 (97.2%) | 36 (100.0%) | 36 (100.0%) | - |

| **OvCa cases and matched controls** | | | | | | | | | | | |
|---|---|---|---|---|---|---|---|---|---|---|---|
| **Rounds** | **Sample types** | **Assays** | **Groups** | **Sample resourses** | **Target N** | **Mean of age (range)** | **Median of age** | **Assayed N (call rate %)** | | | |
| | | | | | | | | ***S100P*** | ***SLC22A18*** | ***DYRK4*** | ***FUT7*** |
| OvCa and controls | DNA from blood pellet | MassARRAY | controls | University hospital of Heidelberg | 148 | 37.9 (21-63) | 38 | 147 (99.3%) | 147 (99.3%) | | 144 (97.3%) |
| | | | sporadic OvCa cases | University hospital of Heidelberg | 84 | 61.8 (37-80) | 61.5 | 78 (92.9%) | 84 (100.0%) | | 82 (97.6%) |

| **PaCa cases and matched controls** | | | | | | | | | | | |
|---|---|---|---|---|---|---|---|---|---|---|---|
| **Rounds** | **Sample types** | **Assays** | **Groups** | **Sample resourses** | **Target N** | **Mean of age (range)** | **Median of age** | **Assayed N (call rate %)** | | | |
| | | | | | | | | ***S100P*** | ***SLC22A18*** | ***DYRK4*** | ***FUT7*** |
| PaCa and controls | Peripheral blood DNA | MassARRAY | controls | Blood donor from Mannheim | 191 | 58.8 (21-68) | 61 | 184 (96.3%) | | | |
| | | | sporadic PaCa cases | | 147 | 62.9 (19-86) | 64 | 133 (90.5%) | | | |

**Table 2. Methylation difference of CpG sites in discovery round (BC)**

| **CpG sites** | **Difference in methylation levels** | | |
|---|---|---|---|
| | **Controls median (IQR)** | **Familial BC cases median (IQR)** | **Adjusted *p* (Illumina)** |
| ***S100P* (cg22266967)** | 0.44 (0.36-0.52) | 0.31 (0.26-0.38) | 2.51E-08 |
| ***SLC22A18* (cg21019522)** | 0.25 (0.24-0.30) | 0.19 (0.16-0.23) | 6.30E-07 |
| ***DYRK4* (cg09418321)** | 0.32 (0.24-0.36) | 0.21 (0.18-0.25) | 8.19E-09 |
| ***FUT7* (cg02679745)** | 0.36 (0.32-0.40) | 0.28 (0.23-0.33) | 7.31 E-07 |

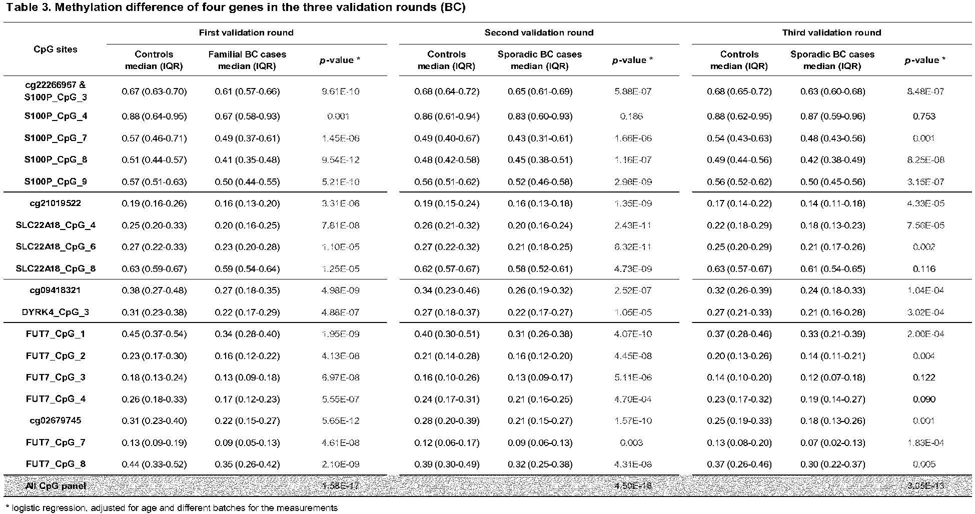

**Table 4. Inter-quartile analysis for the methylation leveles of four genes in the first validation round (BC)**

| **CpG sites** | **Quartiles (methylation range)** | **Controls N (%)** | **Familial BC cases N (%)** | **OR (95% Cl)*** | ***p*-value*** |
|---|---|---|---|---|---|
| **cg22266967 & S100P_CpG_3** | Q4 (> 68%) | 57 (35.6%) | 22 (13.8%) | 1.00 | - |
| | Q3 (≤ 68%) | 45 (28.1%) | 31 (19.4%) | 1.86 (0.94-3.68) | 0.074 |
| | 02 (≤ 64%) | 37 (23.1%) | 36 (22.5%) | 2.72 (1.37-5.39) | 0.004 |
| | Q1 (≤ 60%) | 21 (13.1%) | 71 (44.4%) | 9.14 (4.52-18.47) | 7.09E-10 |
| **S100P_CpG_4** | Q4 (> 94%) | 43 (26.9%) | 31 (19.4%) | 1.00 | - |
| | Q3 (≤ 94%) | 49 (30.6%) | 35 (21.9%) | 0.99 (0.52-1.89) | 0.974 |
| | Q2 (≤ 84%) | 43 (26.9%) | 36 (22.5%) | 1.20 (0.63-2.29) | 0.574 |
| | Q1 (≤ 60%) | 25 (15.6%) | 57 (35.6%) | 3.23 (1.66-6.29) | 0.001 |
| **S100P_CpG_7** | Q4 (> 65%) | 50 (31.3%) | 27 (16.9%) | 1.00 | - |
| | Q3 (≤ 65%) | 42 (26.3%) | 36 (22.5%) | 1.63 (0.85-3.13) | 0.140 |
| | Q2 (≤ 53%) | 40 (25.0%) | 43 (26.9%) | 2.19 (1.14-4.21) | 0.019 |
| | Q1 (≤ 41%) | 28 (17.5%) | 53 (33.1%) | 5.92 (2.7-12.99) | 8.93E-06 |
| **S100P_CpG_8** | Q4 (> 53%) | 62 (38.8%) | 14 (8.8%) | 1.00 | - |
| | Q3 (≤ 53%) | 47 (29.4%) | 36 (22.5%) | 3.81 (1.81-8.03) | 4.34E-04 |
| | Q2 (≤ 46%) | 35 (21.9%) | 44 (27.5%) | 6.52 (3.05-13.92) | 1.29E-06 |
| | Q1 (≤ 39%) | 16 (10.0%) | 65 (40.6%) | 20.53 (9.00-46.82) | 6.77E-13 |
| **S100P_CpG_9** | Q4 (> 60%) | 52 (32.5%) | 16 (10.0%) | 1.00 | - |
| | Q3 (≤ 60%) | 52 (32.5%) | 29 (18.1%) | 1.86 (0.88-3.91) | 0.101 |
| | Q2 (≤ 53%) | 38 (23.8%) | 52 (32.5%) | 5.00 (2.43-10.30) | 1.23E-05 |
| | Q1 (≤ 47%) | 18 (11.3%) | 62 (38.8%) | 12.36 (5.61-27.24) | 4.36E-10 |
| **cg21019522** | Q4 (> 22%) | 54 (33.8%) | 21 (13.1%) | 1.00 | - |
| | Q3 (≤ 22%) | 31 (19.4%) | 28 (17.5%) | 2.33 (1.13-4.82) | 0.023 |
| | Q2 (≤ 18%) | 41 (25.6%) | 54 (33.8%) | 3.41 (1.77-6.58) | 2.49E-04 |
| | Q1 (≤ 14%) | 29 (18.1%) | 54 (33.8%) | 4.72 (2.38-9.36) | 9.28E-06 |
| **SLC22A18_CpG_4** | Q4 (> 29%) | 54 (33.8%) | 21 (13.1%) | 1.00 | - |
| | Q3 (≤ 29%) | 36 (22.5%) | 29 (18.1%) | 1.99 (0.98-4.06) | 0.057 |
| | Q2 (≤ 23%) | 33 (20.6%) | 43 (26.9%) | 3.3 (1.67-6.52) | 0.001 |
| | Q1 (≤ 18%) | 33 (20.6%) | 65 (40.6%) | 5.08 (2.6-9.94) | 2.03E-06 |
| **SLC22A18_CpG_6** | Q4 (> 31%) | 43 (26.9%) | 23 (14.4%) | 1.00 | - |
| | Q3 (≤ 31%) | 46 (28.8%) | 36 (22.5%) | 1.58 (0.80-3.12) | 0.189 |
| | Q2 (≤ 25%) | 39 (24.4%) | 44 (27.5%) | 2.24 (1.14-4.4) | 0.019 |
| | Q1 (≤ 21%) | 28 (17.5%) | 55 (34.4%) | 3.81 (1.90-7.63) | 1.58E-04 |
| **SLC22A18_CpG_8** | Q4 (> 65%) | 50 (31.3%) | 24 (15.0%) | 1.00 | - |
| | Q3 (≤ 65%) | 40 (25.0%) | 31 (19.4%) | 1.52 (0.77-3.01) | 0.229 |
| | Q2 (≤ 61%) | 41 (25.6%) | 47 (29.4%) | 2.28 (1.20-4.36) | 0.012 |
| | Q1 (≤ 56%) | 25 (15.6%) | 55 (34.4%) | 4.34 (2.19-8.63) | 2.77E-05 |

| | | | | | |
|---|---|---|---|---|---|
| * logistic regression, adjusted for age and different batches for the measurements | | | | | |

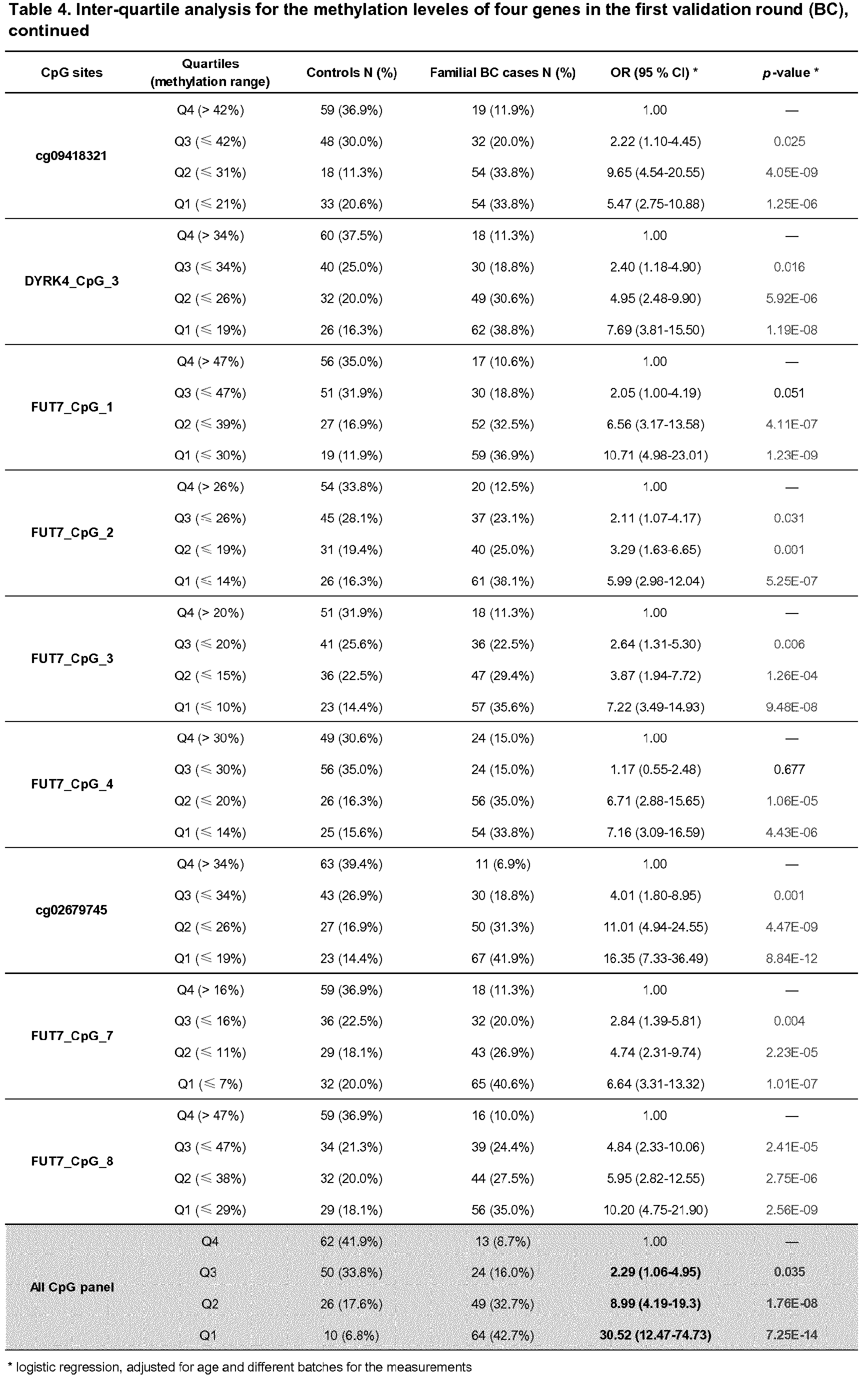

**Table 5. Inter-quartile analysis for the methylation leveles of four genes in the second validation round (BC)**

| **CpG sites** | **Quartiles (methylation range)** | **Controls N (%)** | **Sporadic BC cases N (%)** | **OR (95% Cl) *** | ***p*-value*** |
|---|---|---|---|---|---|
| **cg22266967 & S100P_CpG_3** | Q4 (> 70%) | 58 (30.7%) | 28 (14.8%) | 1.00 | - |
| | Q3 (≤ 70%) | 51 (27.0%) | 43 (22.8%) | 1.80 (0.98-3.32) | 0.059 |
| | Q2 (≤ 66%) | 43 (22.8%) | 43 (22.8%) | 2.10 (1.13-3.90) | 0.019 |
| | Q1 (≤ 63%) | 37 (19.6%) | 74 (39.2%) | 4.40 (2.39-8.07) | 1.81 E-06 |
| **S100P_CpG_4** | Q4 (> 93%) | 51 (27.0%) | 37 (19.6%) | 1.00 | - |
| | Q3 (≤ 93%) | 47 (24.9%) | 51 (27.0%) | 1.56 (0.87-2.81) | 0.134 |
| | Q2 (≤ 84%) | 41 (21.7%) | 42 (22.2%) | 1.45 (0.79-2.67) | 0.228 |
| | Q1 (≤ 61%) | 50 (26.5%) | 57 (30.2%) | 1.68 (0.93-3.01) | 0.083 |
| **S100P_CpG_7** | Q4 (> 63%) | 52 (27.5%) | 37 (19.6%) | 1.00 | - |
| | Q3 (≤ 63%) | 52 (27.5%) | 43 (22.8%) | 1.48 (0.81-2.72) | 0.203 |
| | Q2 (≤ 47%) | 49 (25.9%) | 38 (20.1%) | 2.28 (1.08-4.80) | 0.030 |
| | Q1 (≤ 36%) | 36 (19.0%) | 69 (36.5%) | 7.65 (3.32-17.60) | 1.73E-06 |
| **S100P_CpG_8** | Q4 (> 53%) | 63 (33.3%) | 28 (14.8%) | 1.00 | - |
| | Q3 (≤ 53%) | 36 (19.0%) | 49 (25.9%) | 3.49 (1.85-6.58) | 1.14E-04 |
| | Q2 (≤ 46%) | 57 (30.2%) | 49 (25.9%) | 2.32 (1.26-4.28) | 0.007 |
| | Q1 (≤ 40%) | 33 (17.5%) | 62 (32.8%) | 5.47 (2.83-10.6) | 4.65E-07 |
| **S100P_CpG_9** | Q4 (> 60%) | 60 (31.7%) | 27 (14.3%) | 1.00 | - |
| | Q3 (≤ 60%) | 49 (25.9%) | 45 (23.8%) | 2.19 (1.18-4.05) | 0.013 |
| | Q2 (≤ 54%) | 49 (25.9%) | 52 (27.5%) | 2.87 (1.54-5.37) | 0.001 |
| | Q1 (≤ 48%) | 31 (16.4%) | 64 (33.9%) | 6.33 (3.19-12.53) | 1.22E-07 |
| **cg21019522** | Q4 (> 21%) | 66 (34.9%) | 15 (7.9%) | 1.00 | - |
| | Q3 (≤ 21%) | 51 (27.0%) | 43 (22.8%) | 3.70 (1.85-7.39) | 2.13E-04 |
| | Q2 (≤ 17%) | 34 (18.0%) | 61 (32.3%) | 8.03 (3.98-16.22) | 6.33E-09 |
| | Q1 (≤ 14%) | 37 (19.6%) | 69 (36.5%) | 8.36 (4.17-16.77) | 2.29E-09 |
| **SLC22A18_CpG_4** | Q4 (> 28%) | 68 (36.0%) | 15 (7.9%) | 1.00 | - |
| | Q3 (≤ 28%) | 54 (28.6%) | 47 (24.9%) | 3.93 (1.98-7.78) | 8.65E-05 |
| | Q2 (≤ 22%) | 39 (20.6%) | 57 (30.2%) | 6.29 (3.14-12.61) | 2.14E-07 |
| | Q1 (≤ 17%) | 27 (14.3%) | 72 (38.1%) | 12.02 (5.88-24.58) | 9.45E-12 |
| **SLC22A18_CpG_6** | Q4 (> 29%) | 66 (34.9%) | 15 (7.9%) | 1.00 | - |
| | Q3 (≤ 29%) | 60 (31.7%) | 46 (24.3%) | 3.44 (1.74-6.80) | 3.96E-04 |
| | Q2 (≤ 23%) | 30 (15.9%) | 62 (32.8%) | 9.68 (4.72-19.87) | 6.07E-10 |
| | Q1 (≤ 19%) | 32 (16.9%) | 65 (34.4%) | 9.31 (4.58-18.94) | 7.06E-10 |
| **SLC22A18_CpG_8** | Q4 (> 64%) | 69 (36.5%) | 19 (10.1%) | 1.00 | - |
| | Q3 (≤ 64%) | 39 (20.6%) | 39 (20.6%) | 3.72 (1.88-7.36) | 1.58E-04 |
| | Q2 (≤ 60%) | 39 (20.6%) | 57 (30.2%) | 5.41 (2.81-10.42) | 4.54E-07 |
| | Q1 (≤ 55%) | 41 (21.7%) | 73 (38.6%) | 6.73 (3.51-12.89) | 9.42E-09 |

| | | | | | |
|---|---|---|---|---|---|
| * logistic regression, adjusted for age and different batches for the measurements | | | | | |

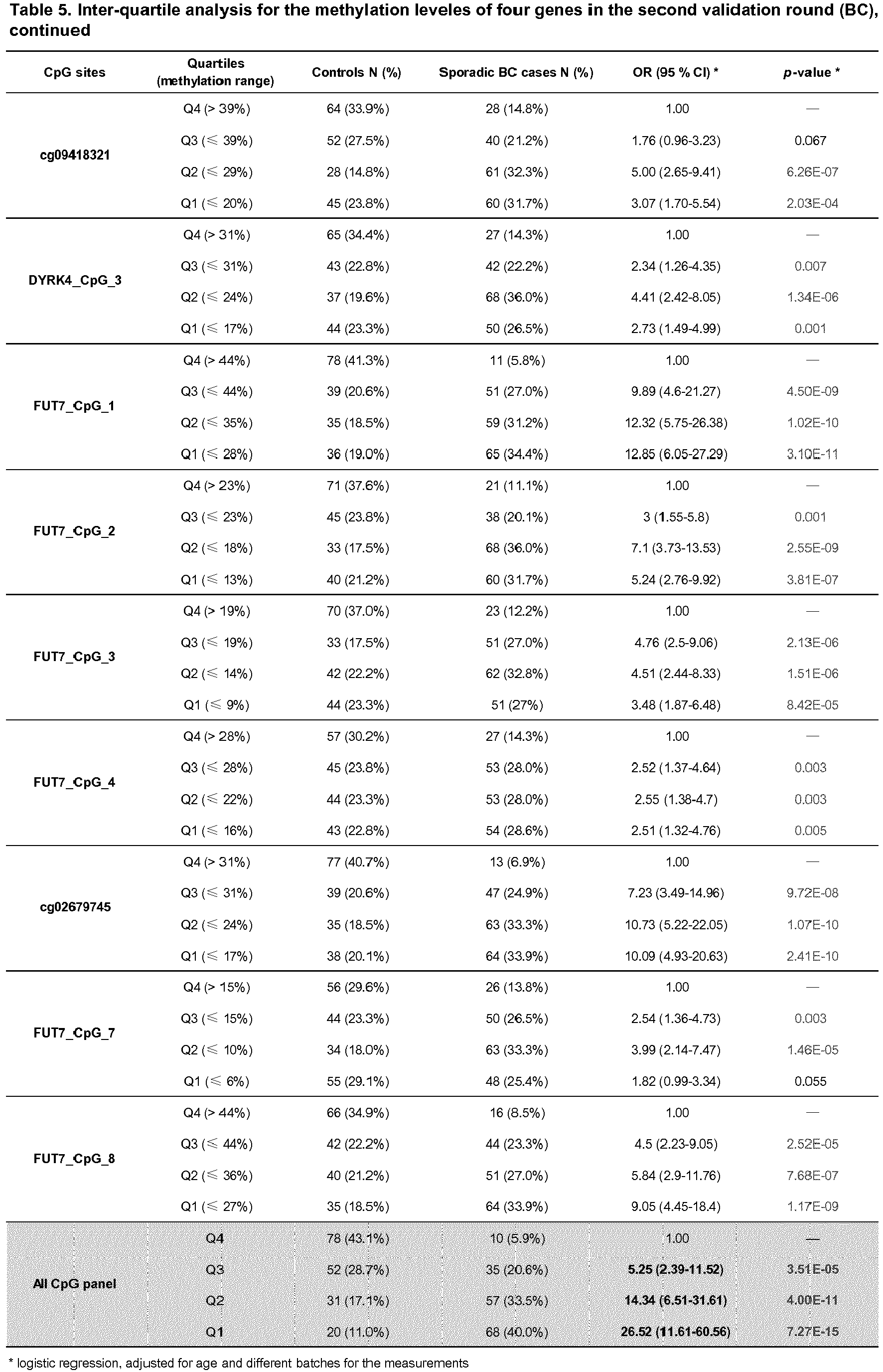

**Table 6. Inter-quartile analysis for the methylation leveles of four genes in the third validation round (BC)**

| **CpG sites** | **Quartiles (methylation range)** | **Controls N (%)** | **Sporadic BC cases N (%)** | **OR (95**% **Cl)** * | ***p*-value** * |
|---|---|---|---|---|---|
| **cg22266967 & S100P_CpG_3** | Q4 (> 71%) | 28 (29.5%) | 9 (9.5%) | 1.00 | - |
| | Q3 (≤ 71%) | 28 (29.5%) | 21 (22.1%) | 2.61 (0.95-7.14) | 0.062 |
| | Q2 (≤ 66%) | 28 (29.5%) | 24 (25.3%) | 4.00 (1.45-11.07) | 0.008 |
| | Q1 (≤ 62%) | 11 (11.6%) | 40 (42.1%) | 17.64 (5.59-55.71) | 9.96E-07 |
| **S100P_CpG_4** | Q4 (> 96%) | 15 (15.8%) | 23 (24.2%) | 1.00 | - |
| | Q3 (≤ 96%) | 32 (33.7%) | 22 (23.2%) | 0.39 (0.16-0.96) | 0.041 |
| | Q2 (≤ 88%) | 26 (27.4%) | 22 (23.2%) | 0.53 (0.21-1.35) | 0.182 |
| | Q1 (≤ 61%) | 22 (23.2%) | 27 (28.4%) | 0.76 (0.30-1.96) | 0.576 |
| **S100P_CpG_7** | Q4 (> 61%) | 29 (30.5%) | 11 (11.6%) | 1.00 | - |
| | Q3 (≤ 61%) | 25 (26.3%) | 25 (26.3%) | 4.34 (1.59-11.84) | 0.004 |
| | Q2 (≤ 51%) | 16 (16.8%) | 26 (27.4%) | 6.57 (2.31-18.72) | 4.18E-04 |
| | Q1 (≤ 43%) | 25 (26.3%) | 32 (33.7%) | 6.76 (2.41-18.91) | 2.74E-04 |
| **S100P_CpG_8** | Q4 (> 53%) | 32 (33.7%) | 13 (13.7%) | 1.00 | - |
| | Q3 (≤ 53%) | 28 (29.5%) | 17 (17.9%) | 2.81 (1.03-7.66) | 0.044 |
| | Q2 (≤ 46%) | 23 (24.2%) | 21 (22.1%) | 4.99 (1.80-13.82) | 0.002 |
| | Q1 (≤ 40%) | 12 (12.6%) | 43 (45.3%) | 20.11 (6.77-59.79) | 6.70E-08 |
| **S100P_CpG_9** | Q4 (> 59%) | 30 (31.6%) | 16 (16.8%) | 1.00 | - |
| | Q3 (≤ 59%) | 30 (31.6%) | 13 (13.7%) | 1.33 (0.49-3.57) | 0.573 |
| | Q2 (≤ 54%) | 21 (22.1%) | 25 (26.3%) | 3.54 (1.38-9.10) | 0.009 |
| | Q1 (≤ 48%) | 14 (14.7%) | 40 (42.1%) | 12.65 (4.43-36.15) | 2.15E-06 |
| **cg21019522** | Q4 (> 20%) | 29 (30.5%) | 11 (11.6%) | 1.00 | - |
| | Q3 (≤ 20%) | 25 (26.3%) | 21 (22.1%) | 3.68 (1.33-10.15) | 0.012 |
| | Q2 (≤ 16%) | 23 (24.2%) | 26 (27.4%) | 5.28 (1.92-14.5) | 0.001 |
| | Q1 (≤ 12%) | 17 (17.9%) | 37 (38.9%) | 7.85 (2.95-20.91) | 3.70E-05 |
| **SLC22A18_CpG_4** | Q4 (> 26%) | 30 (31.6%) | 13 (13.7%) | 1.00 | - |
| | Q3 (≤ 26%) | 24 (25.3%) | 20 (21.1%) | 2.62 (0.99-6.92) | 0.052 |
| | Q2 (≤ 20%) | 26 (27.4%) | 27 (28.4%) | 3.11 (1.23-7.82) | 0.016 |
| | Q1 (≤ 15%) | 14 (14.7%) | 35 (36.8%) | 7.58 (2.84-20.18) | 5.09E-05 |
| **SLC22A18_CpG_6** | Q4 (> 28%) | 26 (27.4%) | 13 (13.7%) | 1.00 | - |
| | Q3 (≤ 28%) | 29 (30.5%) | 18 (18.9%) | 1.91 (0.72-5.06) | 0.192 |
| | Q2 (≤ 23%) | 20 (21.1%) | 29 (30.5%) | 4.32 (1.63-11.44) | 0.003 |
| | Q1 (≤ 18%) | 19 (20.0%) | 35 (36.8%) | 5.62 (2.12-14.89) | 0.001 |
| **SLC22A18_CpG_8** | Q4 (> 67%) | 23 (24.2%) | 14 (14.7%) | 1.00 | - |
| | Q3 (≤ 67%) | 32 (33.7%) | 25 (26.3%) | 1.64 (0.65-4.12) | 0.295 |
| | Q2 (≤ 61%) | 17 (17.9%) | 22 (23.2%) | 1.98 (0.73-5.35) | 0.178 |
| | Q1 (≤ 56%) | 22 (23.2%) | 34 (35.8%) | 2.60 (1.02-6.60) | 0.044 |

| | | | | | |
|---|---|---|---|---|---|
| * logistic regression, adjusted for age and different batches for the measurements | | | | | |

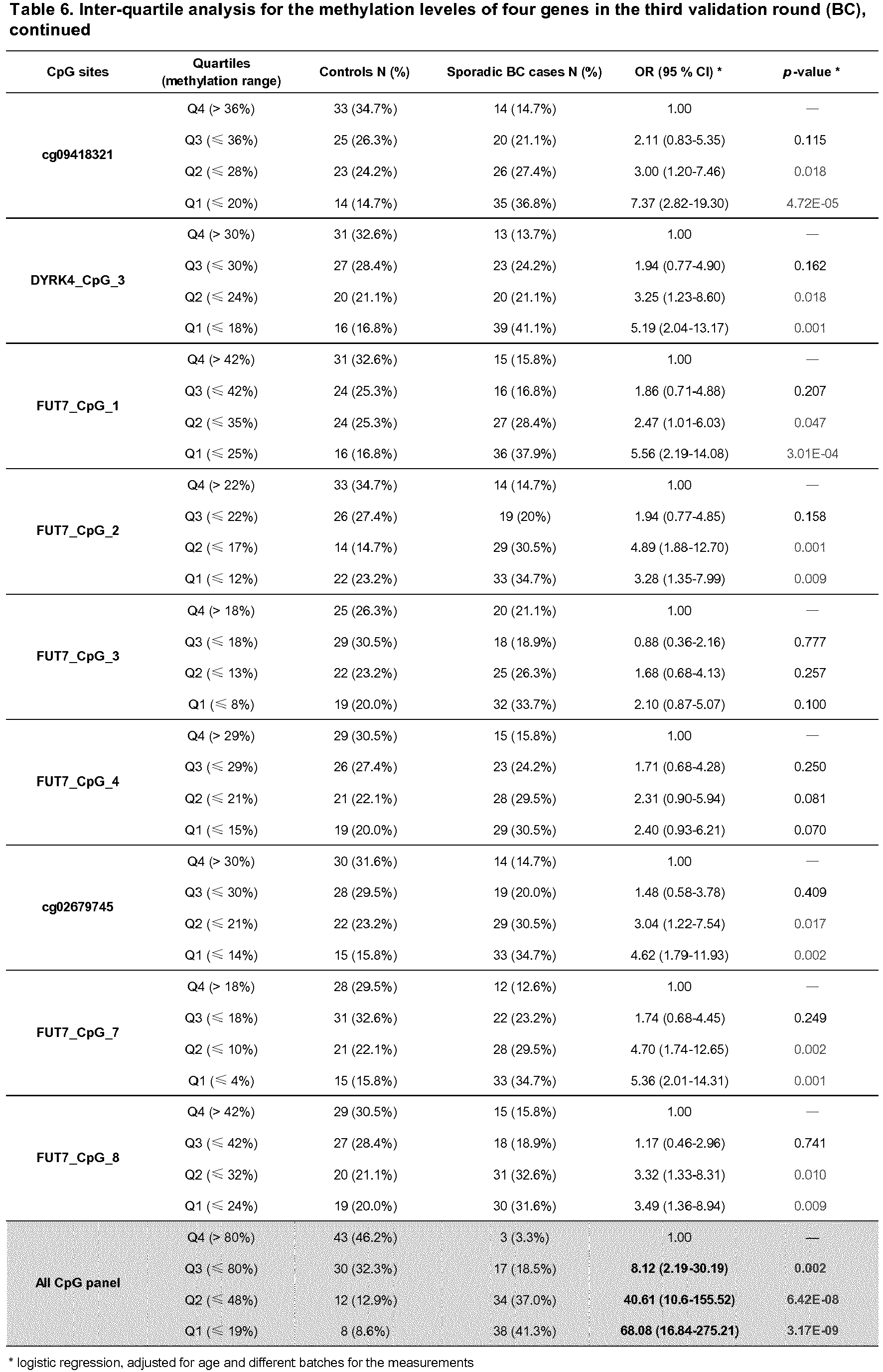

**Table 7. The correlation between methylation and expression of the four genes in leucocytes (BC)**

| **CpG sites** | **Difference in methylation or expression levels** | | | **Correlations to expression** | |
|---|---|---|---|---|---|
| | **Controls median (IQR)** | **Sporadic BC cases median (IQR)** | ***p*-value *** | **Spearman rho** | ***p*-value** |
| **cg22266967 & S100P_CpG_3** | 0.63 (0.58-0.65) | 0.59 (0.56-0.62) | 0.006 | -0.551 | 1.12E-06 |
| **S100P_CpG_4** | 0.60 (0.55-0.87) | 0.61 (0.53-0.83) | 0.445 | -0.147 | 0.226 |
| **S100P_CpG_7** | 0.36 (0.30-0.40) | 0.29 (0.24-0.33) | 0.001 | -0.501 | 8.55E-06 |
| **S100P_CpG_8** | 0.44 (0.38-0.48) | 0.35 (0.32-0.39) | 7.76E-06 | -0.653 | 9.27E-10 |
| **S100P_CpG_9** | 0.49 (0.44-0.54) | 0.45 (0.40-0.47) | 0.001 | -0.555 | 5.07E-07 |
| **Relative expression of S100P** | 1.31 (0.72-2.07) | 3.47 (1.23-20.77) | 0.001 | 1.000 | - |
| **cg21019522** | 0.19 (0.15-0.23) | 0.16 (0.14-0.19) | 0.006 | -0.507 | 5.53E-06 |
| **SLC22A18_CpG_4** | 0.26 (0.20-0.31) | 0.21 (0.17-0.24) | 0.006 | -0.536 | 1.21 E-06 |
| **SLC22A18_CpG_6** | 0.25 (0.20-0.28) | 0.19 (0.17-0.23) | 0.001 | -0.565 | 2.29E-07 |
| **SLC22A18_CpG_8** | 0.64 (0.60-0.70) | 0.59 (0.53-0.63) | 0.001 | -0.525 | 2.15E-06 |
| **Relative expression of SLC22A18** | 0.69 (0.55-0.87) | 0.76 (0.54-1.23) | 0.311 | 1.000 | - |
| **cg09418321** | 0.33 (0.25-0.41) | 0.26 (0.20-0.33) | 0.018 | -0.074 | 0.540 |
| **DYRK4_CpG_3** | 0.29 (0.22-0.34) | 0.24 (0.18-0.28) | 0.023 | -0.075 | 0.536 |
| **Relative expression of DYRK4** | 0.36 (0.31-0.41) | 0.37 (0.29-0.46) | 0.919 | 1.000 | - |

| | | | | | |
|---|---|---|---|---|---|
| *Mann-Whitney U test | | | | | |

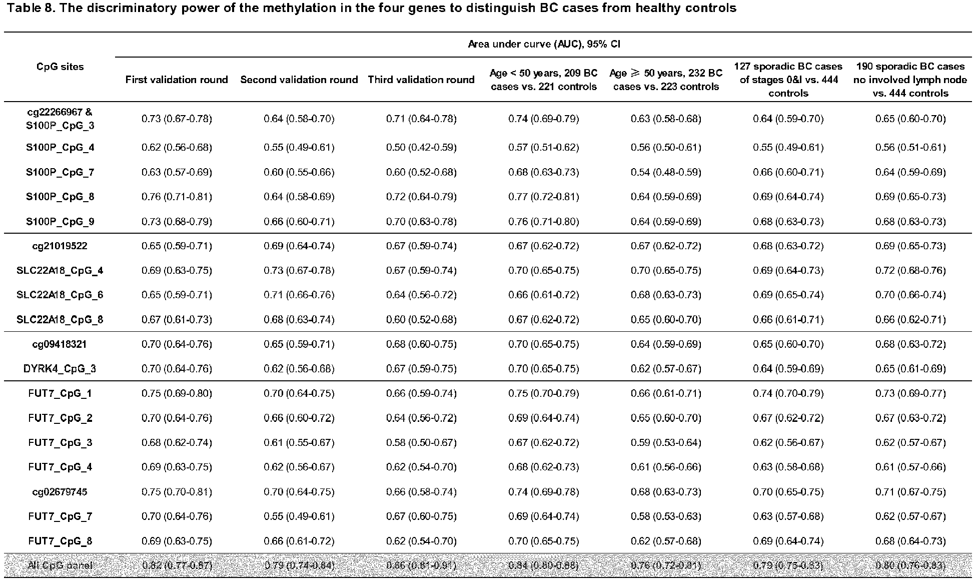

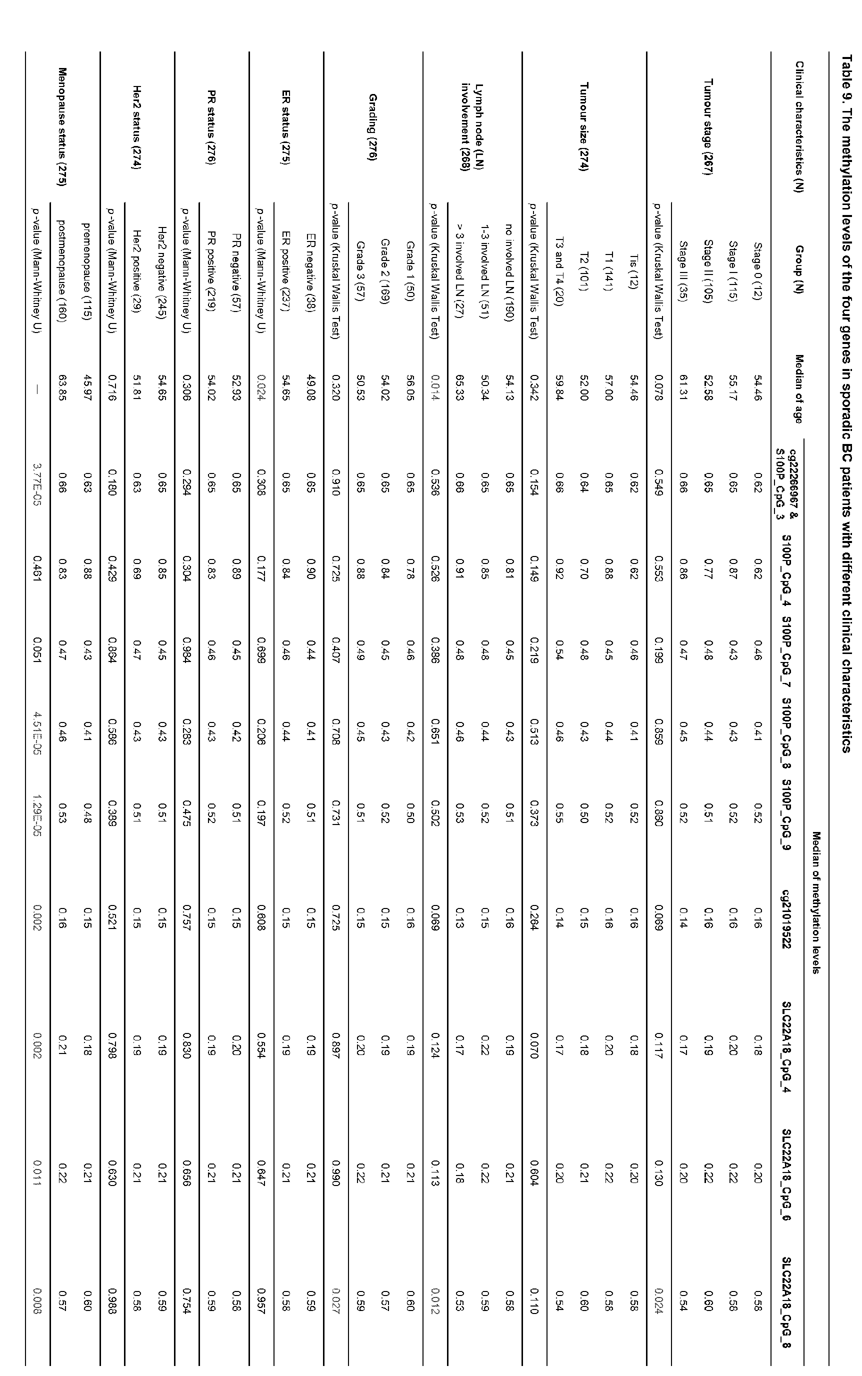

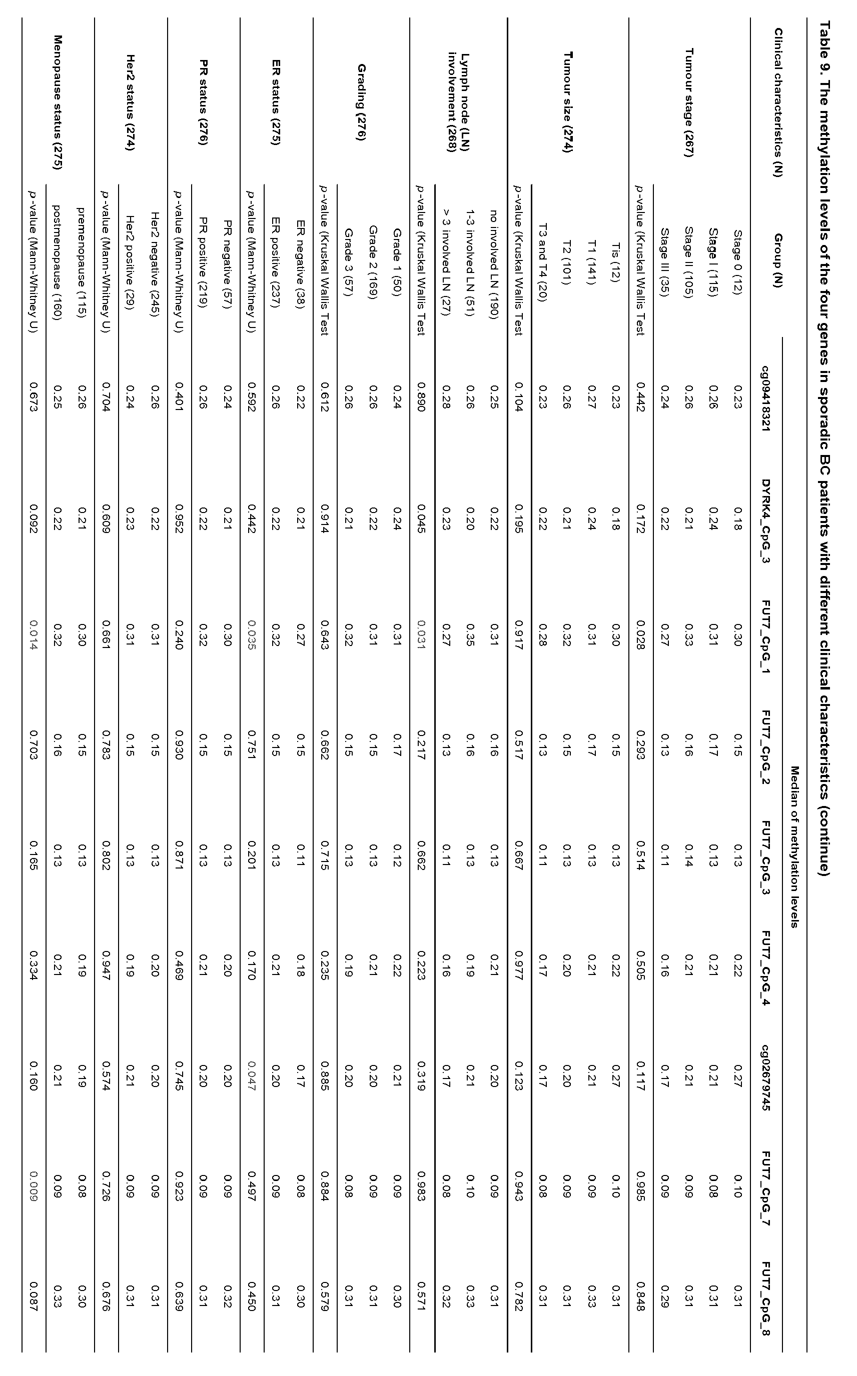

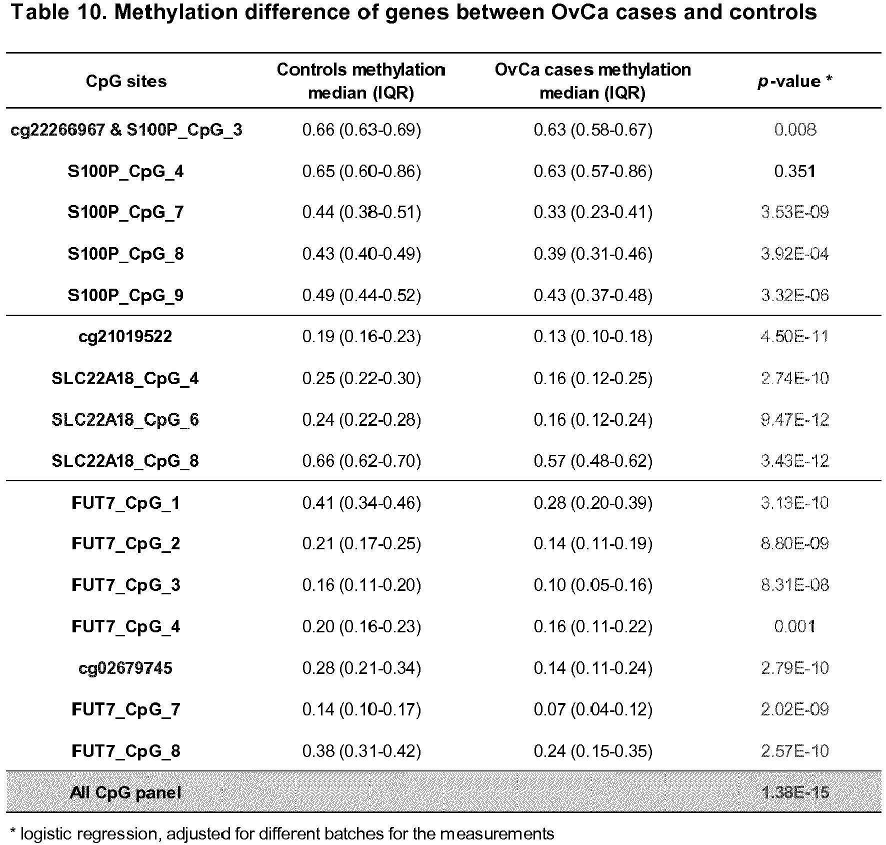

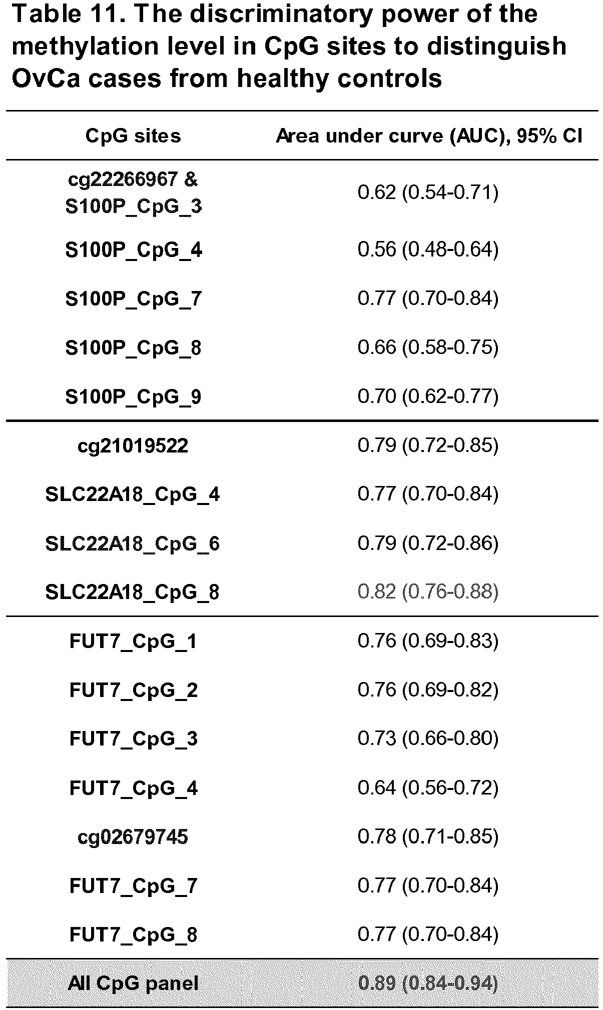

**Table 12. Methylation difference of S100P between PaCa cases and controls**

| **CpG sites** | **All samples** | | | **Male samples** | | | **Female samples** | | |
|---|---|---|---|---|---|---|---|---|---|
| | **Controls median (IQR)** | **PaCa cases median (IQR)** | ***p*-value *** | **Male controls median (IQR)** | **Male PaCa cases median (IQR)** | ***p*-value *** | **Female controls median (IQR)** | **Female PaCa cases median (IQR)** | ***p*-value *** |
| **cg22266967 & S100P_CpG_3** | 0.73 (0.69-0.77) | 0.60 (0.55-0.63) | 9.45E-20 | 0.74 (0.69-0.77) | 0.59 (0.55-0.62) | 1.57E-11 | 0.72 (0.68-0.75) | 0.60 (0.54-0.65) | 1.04E-08 |
| **S100P_CpG_4** | 0.75 (0.66-0.90) | 0.61 (0.55-0.82) | 5.46E-09 | 0.83 (0.67-0.91) | 0.61 (0.56-0.81) | 8.87E-08 | 0.69 (0.63-0.89) | 0.61 (0.52-0.83) | 0.005 |
| **S100P_CpG_7** | 0.64 (0.55-0.74) | 0.32 (0.22-0.43) | 5.49E-20 | 0.65 (0.59-0.76) | 0.30 (0.21-0.42) | 1.38E-10 | 0.60 (0.51-0.68) | 0.33 (0.22-0.45) | 3.45E-09 |
| **S100P_CpG_8** | 0.54 (0.47-0.61) | 0.33 (0.25-0.40) | 2.99E-18 | 0.56 (0.49-0.63) | 0.32 (0.24-0.37) | 4.40E-11 | 0.51 (0.46-0.57) | 0.37 (0.26-0.44) | 2.70E-07 |
| **S100P_CpG_9** | 0.62 (0.56-0.68) | 0.44 (0.37-0.50) | 2.17E-19 | 0.64 (0.58-0.68) | 0.43 (0.37-0.47) | 7.82E-11 | 0.59 (0.54-0.66) | 0.45 (0.38-0.53) | 1.10E-07 |

| | | | | | | | | | |
|---|---|---|---|---|---|---|---|---|---|
| * logistic regression, adjusted for age and different batches for the measurements | | | | | | | | | |

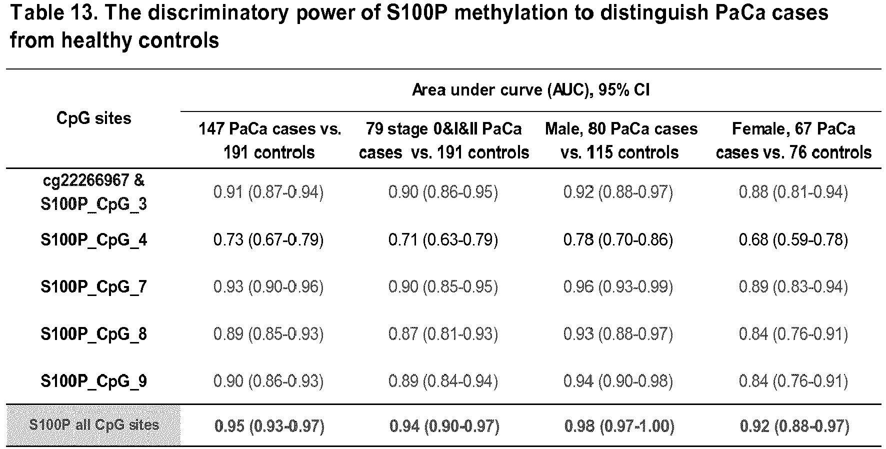

**Table 14. The methylation of genes in PaCa patients with different clinical characteristics**

| **Clinical characteristics (N)** | **Group (N)** | **Median of age** | **Median of methylation levels** | | | | |
|---|---|---|---|---|---|---|---|
| | | | **cg22266967 & S100P_CpG_3** | **S100P_CpG_4** | **S100P_CpG_7** | **S100P_CpG_8** | **S100P_CpG_9** |
| **Tumour stage (97)** | Stage 0 and Stage I (8) | 66.53 | 0.61 | 0.58 | 0.41 | 0.34 | 0.46 |
| | Stage II (71) | 62.86 | 0.59 | 0.62 | 0.33 | 0.34 | 0.44 |
| | Stage III and Stage IV (18) | 68.89 | 0.60 | 0.61 | 0.31 | 0.36 | 0.45 |
| | *p*-value (Kruskal Wallis Test) | 0.410 | 0.818 | 0.799 | 0.989 | 0.501 | 0.831 |
| **Tumour (98)** | < T3 (10) | 63.67 | 0.61 | 0.58 | 0.41 | 0.34 | 0.46 |
| | T3 (77) | 63.64 | 0.59 | 0.61 | 0.32 | 0.33 | 0.43 |
| | T4(11) | 67.55 | 0.60 | 0.61 | 0.32 | 0.36 | 0.47 |
| | *p*-value (Kruskal Wallis Test) | 0.594 | 0.591 | 0.879 | 0.818 | 0.466 | 0.624 |
| **Lymph node involvement (99)** | N0 (28) | 63.67 | 0.60 | 0.60 | 0.33 | 0.32 | 0.44 |
| | N1 (71) | 63.92 | 0.60 | 0.62 | 0.33 | 0.35 | 0.44 |
| | *p*-value (Mann-Whitney Test) | 0.907 | 0.397 | 0.241 | 0.800 | 0.353 | 0.353 |
| **metastasis status (111)** | M0 (91) | 63.56 | 0.60 | 0.61 | 0.33 | 0.35 | 0.44 |
| | M1 (20) | 67.84 | 0.59 | 0.61 | 0.32 | 0.31 | 0.44 |
| | *p*-value (Mann-Whitney Test) | 0.199 | 0.811 | 0.875 | 0.728 | 0.531 | 0.652 |
| **Grading (83)** | Grade 1 & Grade 2 (53) | 64.20 | 0.60 | 0.62 | 0.35 | 0.35 | 0.45 |
| | Grade 3 (30) | 63.41 | 0.61 | 0.61 | 0.32 | 0.36 | 0.46 |
| | *p*-value (Mann-Whitney Test) | 0.798 | 0.686 | 0.707 | 0.623 | 0.392 | 0.808 |
| **Gender (147)** | Male (80) | 63.50 | 0.59 | 0.61 | 0.30 | 0.32 | 0.43 |
| | Female (67) | 66.33 | 0.60 | 0.61 | 0.33 | 0.37 | 0.45 |
| | *p*-value (Mann-Whitney Test) | 0.605 | 0.377 | 0.749 | 0.250 | 0.062 | 0.339 |

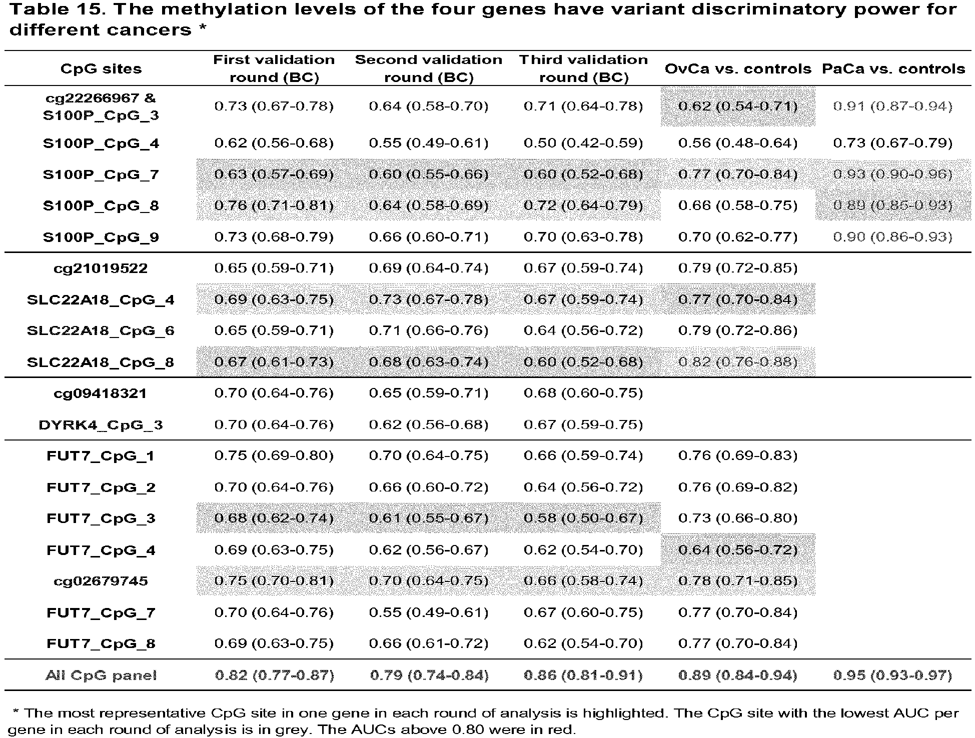

**Table 16 The methylation levels of S100P CpG sites by Illumina 450K**

| **CpG ID** | **pvalWald_Group** | **meanDiff** | **BUILD** | **CHR** | **MAPINFO** | **LOCATION** | **ENHANCER** |
|---|---|---|---|---|---|---|---|
| cg02883621 | 0.6312 | -0.003 | 36 | 4 | 6745824 | TSS1500 | NA |
| cg14323984 | 0.7019 | -0.002 | 36 | 4 | 6746104 | TSS1500 | NA |
| cg27027375 | 0.0233 | -0.017 | 36 | 4 | 6746220 | TSS1500 | NA |
| cg14900031 | 0.0010 | -0.021 | 36 | 4 | 6746278 | TSS200 | NA |
| cg14140379 | 0.0011 | -0.029 | 36 | 4 | 6746281 | TSS200 | NA |
| cg25083732 | 0.0534 | -0.027 | 36 | 4 | 6746365 | TSS200 | NA |
| cg07210669 | 0.0117 | -0.028 | 36 | 4 | 6746376 | TSS200 | NA |
| cg26233331 | 0.0002 | -0.040 | 36 | 4 | 6746515 | 1stExon;5'UTR | NA |
| cg22266967 | 0.0014 | -0.033 | 36 | 4 | 6746599 | 1stExon | NA |
| cg02104700 | 0.3792 | -0.003 | 36 | 4 | 6749069 | Body | NA |

| | | | | | | | |
|---|---|---|---|---|---|---|---|
| a. The CpG sites investigated 450K and MassARRAY were in red. b. The p-values < 0.05 in the column of 'pvalWald_Group' were in red c. The differences of methylation > 0.01 in column 'meanDiff' were in blue. | | | | | | | |

**Table 17 The methylation levels of SLC22A18 CpG sites by Illumina 450K**

| **CpG ID** | **pvalWald_Group** | **meanDiff** | **BUILD** | **CHR** | **MAPINFO** | **LOCATION** | **ENHANCER** |
|---|---|---|---|---|---|---|---|
| cg23335134 | 0.86410 | 0.00117 | 36 | 11 | 2866266 | Body | NA |
| cg26874323 | 0.63776 | 0.00068 | 36 | 11 | 2866285 | Body | NA |
| cg08222610 | 0.08326 | -0.00087 | 36 | 11 | 2866291 | Body | NA |
| cg12240761 | 0.65500 | 0.00247 | 36 | 11 | 2869910 | Body | TRUE |
| cg14449910 | 0.12982 | -0.00776 | 36 | 11 | 2876265 | Body;TSS1500 | TRUE |
| cg26665035 | 0.04257 | -0.02301 | 36 | 11 | 2876339 | Body;TSS1500 | TRUE |
| cg22040301 | 0.24166 | -0.01200 | 36 | 11 | 2876374 | Body;TSS1500 | TRUE |
| cg05457684 | 0.05624 | -0.01742 | 36 | 11 | 2876384 | Body;TSS1500 | TRUE |
| cg18419977 | 0.81102 | -0.00021 | 36 | 11 | 2876628 | Body;TSS1500 | TRUE |
| cg24033661 | 0.45850 | -0.00048 | 36 | 11 | 2876631 | Body;TSS1500 | TRUE |
| cg13485320 | 0.65191 | 0.00331 | 36 | 11 | 2876704 | Body;TSS1500 | TRUE |
| cg21853021 | 0.95887 | 0.00213 | 36 | 11 | 2876722 | Body;TSS1500 | TRUE |
| cg18458509 | 0.01700 | -0.01856 | 36 | 11 | 2876765 | Body;TSS1500 | TRUE |
| cg23190089 | 0.00473 | -0.01928 | 36 | 11 | 2876785 | Body;TSS1500 | TRUE |
| cg16587707 | 0.00037 | 0.02235 | 36 | 11 | 2876841 | Body;TSS1500 | TRUE |
| cg02462487 | 0.04036 | 0.01468 | 36 | 11 | 2876926 | Body;TSS1500 | TRUE |
| cg16129800 | 0.00075 | -0.02325 | 36 | 11 | 2876990 | Body;TSS1500 | TRUE |
| cg21599100 | 0.00183 | -0.02196 | 36 | 11 | 2877013 | Body;TSS1500 | TRUE |
| cg05752118 | 0.03104 | -0.01576 | 36 | 11 | 2877140 | Body;TSS1500 | TRUE |
| cg11785933 | 0.00229 | -0.04089 | 36 | 11 | 2877193 | Body;TSS1500 | NA |
| cg25427871 | 0.01368 | -0.03248 | 36 | 11 | 2877311 | Body;TSS1500 | NA |
| cg22315192 | 0.00440 | -0.01563 | 36 | 11 | 2877341 | TSS200;Body | NA |
| cg21019522 | 0.00421 | -0.01841 | 36 | 11 | 2877365 | TSS200;Body | NA |
| cg16346422 | 0.00007 | -0.04249 | 36 | 11 | 2877395 | TSS200;Body | NA |
| cg16873863 | 0.11476 | -0.00938 | 36 | 11 | 2877752 | 5'UTR;5'UTR | NA |
| cg22680591 | 0.57778 | -0.00213 | 36 | 11 | 2878627 | 5'UTR;5'UTR;TSS1500 | NA |
| cg15904130 | 0.16531 | 0.00324 | 36 | 11 | 2878639 | 5'UTR;5'UTR;TSS1500 | NA |
| cg25073813 | 0.02759 | -0.00779 | 36 | 11 | 2878644 | 5'UTR;5'UTR;TSS1500 | NA |
| cg24205453 | 0.41110 | -0.00081 | 36 | 11 | 2878910 | 5'UTR;5'UTR;TSS1500 | NA |
| cg18471235 | 0.00552 | 0.01122 | 36 | 11 | 2878971 | 5'UTR;5'UTR;TSS1500 | NA |
| cg15739881 | 0.09660 | -0.00770 | 36 | 11 | 2879040 | 5'UTR;5'UTR;TSS1500 | NA |
| cg12563184 | 0.24460 | -0.00447 | 36 | 11 | 2879067 | 5'UTR;5'UTR;TSS1500 | NA |
| cg04665867 | 0.34144 | -0.00268 | 36 | 11 | 2879093 | 5'UTR;5'UTR;TSS1500 | NA |
| cg24041239 | 0.13914 | -0.00649 | 36 | 11 | 2879211 | 5'UTR;5'UTR;TSS1500 | NA |
| cg09198782 | 0.30670 | -0.00392 | 36 | 11 | 2879338 | 5'UTR;5'UTR;TSS1500 | NA |
| cg07291601 | 0.46829 | -0.00245 | 36 | 11 | 2879383 | 5'UTR;5'UTR;TSS1500 | NA |
| cg25548316 | 0.79937 | 0.00080 | 36 | 11 | 2879388 | 5'UTR;5'UTR;TSS1500 | NA |
| cg10943932 | 0.50818 | 0.00043 | 36 | 11 | 2879409 | 5'UTR;5'UTR;TSS1500 | NA |
| cg02081198 | 0.63991 | -0.00015 | 36 | 11 | 2879421 | 5'UTR;5'UTR;TSS1500 | NA |
| cg05385260 | 0.05107 | -0.00491 | 36 | 11 | 2879428 | 5'UTR;5'UTR;TSS1500 | NA |
| cg16035277 | 0.47441 | 0.00501 | 36 | 11 | 2879840 | 5'UTR;5'UTR;TSS1500 | NA |
| cg09781437 | 0.01283 | 0.00245 | 36 | 11 | 2879986 | 5'UTR;5'UTR;TSS200 | NA |
| cg02200456 | 0.42924 | -0.00078 | 36 | 11 | 2880012 | 5'UTR;5'UTR;TSS200 | NA |
| cg13671930 | 0.60335 | 0.00278 | 36 | 11 | 2880014 | 5'UTR;5'UTR;TSS200 | NA |
| cg22132309 | 0.71419 | 0.00149 | 36 | 11 | 2880066 | 5'UTR;5'UTR;TSS200 | NA |
| cg16184736 | 0.20026 | -0.00102 | 36 | 11 | 2880074 | 5'UTR;5'UTR;TSS200 | NA |
| cg03829241 | 0.45428 | 0.00125 | 36 | 11 | 2880080 | 5'UTR;5'UTR;TSS200 | NA |
| cg17992161 | 0.08500 | 0.00567 | 36 | 11 | 2880101 | 5'UTR;1stExon;5'UTR;5'UTR | NA |
| cg12733707 | 0.08456 | 0.00468 | 36 | 11 | 2880123 | 5'UTR;1stExon;5'UTR;5'UTR | NA |
| cg24139421 | 0.99706 | 0.00136 | 36 | 11 | 2880154 | 5'UTR;1stExon;5'UTR;5'UTR | NA |
| cg06211616 | 0.89775 | 0.00151 | 36 | 11 | 2880235 | 5'UTR;5'UTR;5'UTR | NA |
| cg24528523 | 0.71569 | 0.00029 | 36 | 11 | 2880384 | 5'UTR;5'UTR;5'UTR | NA |
| cg12911952 | 0.03883 | -0.00749 | 36 | 11 | 2881099 | 5'UTR;5'UTR;5'UTR | NA |
| cg02719634 | 0.09322 | -0.01893 | 36 | 11 | 2881475 | 1stExon;Body;5'UTR;Body | TRUE |
| cg15729154 | 0.03641 | -0.01808 | 36 | 11 | 2881602 | 1stExon;Body;5'UTR;Body | TRUE |
| cg07161669 | 0.26049 | -0.00009 | 36 | 11 | 2881763 | Body;Body;TSS200 | TRUE |
| cg24724917 | 0.48196 | -0.00202 | 36 | 11 | 2882015 | Body;Body;TSS1500 | TRUE |
| cg06495763 | 0.87212 | -0.00122 | 36 | 11 | 2882046 | Body;Body;TSS1500 | TRUE |
| cg06048910 | 0.20155 | -0.00084 | 36 | 11 | 2882049 | Body;Body;TSS1500 | TRUE |
| cg08472797 | 0.27778 | -0.00279 | 36 | 11 | 2882146 | Body;Body;TSS1500 | TRUE |
| cg14101500 | 0.46858 | 0.00042 | 36 | 11 | 2882170 | Body;Body;TSS1500 | TRUE |
| cg22833478 | 0.56017 | -0.00144 | 36 | 11 | 2882199 | Body;Body;TSS1500 | TRUE |
| cg26137286 | 0.05217 | -0.00587 | 36 | 11 | 2882210 | Body;Body;TSS1500 | TRUE |
| cg02390725 | 0.00401 | -0.01006 | 36 | 11 | 2882344 | Body;Body;TSS1500 | TRUE |
| cg20716202 | 0.07785 | -0.00520 | 36 | 11 | 2882423 | Body;Body;TSS1500 | TRUE |
| cg08999895 | 0.25478 | -0.00542 | 36 | 11 | 2882445 | Body;Body;TSS1500 | TRUE |
| cg16530128 | 0.57401 | -0.00230 | 36 | 11 | 2882527 | Body;Body;TSS1500 | NA |
| cg08827700 | 0.00453 | -0.00760 | 36 | 11 | 2882545 | Body;Body;TSS1500 | NA |
| cg22272492 | 0.00094 | -0.01003 | 36 | 11 | 2882572 | Body;Body;TSS1500 | NA |
| cg21991825 | 0.67729 | 0.00152 | 36 | 11 | 2882719 | Body;Body;TSS1500 | NA |
| cg23912877 | 0.61749 | -0.00043 | 36 | 11 | 2882913 | Body;Body;TSS1500 | NA |
| cg06981073 | 0.51912 | -0.00126 | 36 | 11 | 2882920 | Body;Body;TSS1500 | NA |
| cg06669405 | 0.01696 | -0.00962 | 36 | 11 | 2882937 | Body;Body;TSS1500 | NA |
| cg09731124 | 0.49943 | -0.00412 | 36 | 11 | 2882998 | Body;Body;TSS1500 | NA |
| cg02025860 | 0.49162 | -0.00203 | 36 | 11 | 2883177 | Body;Body;TSS1500 | NA |
| cg14168614 | 0.66371 | -0.00466 | 36 | 11 | 2883823 | Body;Body | NA |
| cg02660089 | 0.91020 | 0.00299 | 36 | 11 | 2886017 | Body;Body | NA |
| cg22858288 | 0.44782 | 0.00211 | 36 | 11 | 2886898 | Body;Body | NA |
| cg04726200 | 0.68599 | -0.00716 | 36 | 11 | 2887061 | Body;Body | NA |
| cg19497444 | 0.70525 | -0.00898 | 36 | 11 | 2887370 | Body;Body | NA |
| cg03336167 | 0.98215 | -0.00545 | 36 | 11 | 2887571 | Body;Body | NA |
| cg23698969 | 0.38340 | -0.00335 | 36 | 11 | 2887741 | Body;Body | NA |
| cg24409566 | 0.69448 | -0.00247 | 36 | 11 | 2890543 | Body;Body | TRUE |
| cg05351334 | 0.63950 | -0.00260 | 36 | 11 | 2896968 | Body;Body | NA |
| cg14275836 | 0.77431 | -0.00245 | 36 | 11 | 2897889 | Body;Body | NA |
| cg19240938 | 0.27819 | 0.00219 | 36 | 11 | 2898612 | Body;Body | NA |
| cg18655584 | 0.99721 | -0.00182 | 36 | 11 | 2898669 | Body;Body | NA |
| cg12510502 | 0.53115 | -0.00235 | 36 | 11 | 2898752 | Body;Body | NA |
| cg13328151 | 0.24586 | 0.00388 | 36 | 11 | 2899615 | Body;Body | NA |
| cg03010425 | 0.95099 | 0.00172 | 36 | 11 | 2901347 | Body;Body | NA |
| cg26595893 | 0.89409 | 0.00165 | 36 | 11 | 2903050 | 3'UTR;3'UTR | NA |

| | | | | | | | |
|---|---|---|---|---|---|---|---|
| a. The CpG sites investigated 450K and MassARRAY were in red. b. The p-values < 0.05 in the column of 'pvalWald_Group' were in red c. The differences of methylation > 0.01 in column 'meanDiff' were in blue. | | | | | | | |

**Table 18 The methylation levels of DYRK4 CpG sites by Illumina 450K**

| **CpG ID** | **pvalWald_Group** | **meanDiff** | **BUILD** | **CHR** | **MAPINFO** | **LOCATION** | **ENHANCER** |
|---|---|---|---|---|---|---|---|
| cg24707294 | 0.80899 | -0.00296 | 36 | 12 | 4568380 | TSS1500 | NA |
| cg08977032 | 0.59206 | -0.00127 | 36 | 12 | 4568578 | TSS1500 | NA |
| cg06270401 | 0.00005 | -0.04711 | 36 | 12 | 4569346 | TSS200 | NA |
| cg09581911 | 0.81051 | -0.01355 | 36 | 12 | 4569493 | TSS200 | NA |
| cg09418321 | 0.00111 | -0.03126 | 36 | 12 | 4569879 | 5'UTR | NA |
| cg01218945 | 0.40781 | 0.00053 | 36 | 12 | 4584370 | Body | NA |
| cg24337818 | 0.05390 | -0.00208 | 36 | 12 | 4584440 | Body | NA |
| cg00532413 | 0.23807 | -0.00023 | 36 | 12 | 4584588 | Body | NA |

| | | | | | | | |
|---|---|---|---|---|---|---|---|
| a. The CpG sites investigated 450K and MassARRAY were in red. b. The p-values < 0.05 in the column of 'pvalWald_Group' were in red c. The differences of methylation > 0.01 in column 'meanDiff' were in blue. | | | | | | | |

**Table 19 The methylation levels of FUT7 CpG sites by Illumina 450K**

| **CpG ID** | **pvalWald_Group** | **meanDiff** | **BUILD** | **CHR** | **MAPINFO** | **LOCATION** | **ENHANCER** |
|---|---|---|---|---|---|---|---|
| cg02971262 | 0.00311 | -0.01121 | 36 | 9 | 139045216 | 5'UTR;Body | NA |
| cg14205519 | 0.58705 | 0.00166 | 36 | 9 | 139045571 | 5'UTR;Body | NA |
| cg03630596 | 0.66317 | -0.00260 | 36 | 9 | 139045677 | 5'UTR;Body | NA |
| cg13757845 | 0.00822 | -0.02277 | 36 | 9 | 139046561 | 5'UTR;1stExon;5'UTR | NA |
| cg09305224 | 0.00013 | -0.03032 | 36 | 9 | 139047066 | 5'UTR;1stExon;5'UTR | NA |
| cg02679745 | 0.00003 | -0.03635 | 36 | 9 | 139047467 | Body;TSS1500 | NA |

| | | | | | | | |
|---|---|---|---|---|---|---|---|
| a. The CpG sites investigated 450K and MassARRAY were in red. b. The p-values < 0.05 in the column of 'pvalWald_Group' were in red c. The differences of methylation > 0.01 in column 'meanDiff' were in blue. | | | | | | | |

## Claims

1. A non-invasive method for early diagnosing cancer or a susceptibility of developing cancer in a subject, comprising:
(a) providing a sample from the subject, wherein the sample comprises DNA, RNA and/or protein;
(b) determining the methylation status and/or the expression level of a panel of genes comprising S100P (S100 calcium binding protein P), SLC22A18 (Solute carrier family 22, member 18), DYRK4 (dual-specificity tyrosine-(Y)-phosphorylation regulated kinase 4) and FUT7 (fucosyltransferase 7), or of at least one of said genes;
wherein an altered methylation and/or expression compared to a corresponding reference value or control sample of a healthy subject is indicative for the presence or risk and susceptibility of development cancer, preferably wherein the altered methylation and/or expression level is a (i) decreased methylation and/or (ii) over-expression compared to a corresponding reference value or control sample of a healthy subject.

2. The method of claim 1, wherein said methylation comprises methylation of at least one CpG site and/or the expression level is determined by measuring the mRNA and/or protein level.

3. The method of claims 1 or 2,
(i) wherein at least the methylation of one CpG site as shown in Figure 1B and/or 6 is determined and/or
(ii) wherein at least the methylation of one or more CpG sites is determined selected from the group consisting of CpG sites cg22266967 in S100P (Chr 4: 6746599, Build 36.1/hg18), cg21019522 in SLC22A18 (Chr 11: 2877365, Build 36.1/hg18), cg09418321 in DYRK4 (Chr 12: 4569879, Build 36.1/hg18) and cg02679745 in FUT7 (Chr 9: 139047467, Build 36.1/hg18).

4. The method of any one of claims 1 to 3, wherein the methylation status of at least 2, at least 3, at least 4, at least 5, at least 6 or at least 7 of said CpG sites is determined, and/or the CpG sites of at least 2, at least 3 or at least 4 of the genes are determined.

5. The method of any one of claims 1 to 4, wherein DNA methylation is determined by using bisulfite-specific primers, preferably selected from the group of primers amplifying and/or comprising or consisting of the nucleotide sequence of S100P (NC_000004.12 Reference GRCh38 Primary Assembly, AC_000136.1 Alternate HuRef, NC_018915.2 Alternate CHM1_1.1), SLC22A18 (NC_000011.10 Reference GRCh38 Primary Assembly, NG_011512.1 RefSeqGene, NT_187585.1 Reference GRCh38 ALT_REF_LOCI_1, AC_000143.1 Alternate HuRef, NC_018922.2 Alternate CHM1_1.1), DYRK4 (NC_000012.12 Reference GRCh38 Primary Assembly, AC_000144.1 Alternate HuRef, NC_018923.2 Alternate CHM1_1.1), FUT 7 (NC_000009.12 Reference GRCh38 Primary Assembly, NG_007527.1 RefSeqGene, AC_000141.1 Alternate HuRef, NC_018920.2 Alternate CHM1_1.1) or fragments thereof or its bisulfite-converted sequence, preferably wherein the primer or primer pair is selected from the group of primers comprising or consisting of the nucleotide sequence of SEQ ID NOs 5 and 6, 7 and 8, 9 and 10, and 11 and 12, respectively.

6. The method of any one of claims 1 to 5, wherein the sample comprises peripheral blood or is derived thereof.

7. The method of any one of claims 1 to 6, wherein
(i) the subject is substantially healthy and does not show any significant symptoms or signs of cancer;
(ii) the age of the subject is < 50; and/or
(iii) the subject is a familiar cancer patient or a sporadic cancer patient.

8. The method of any one of claims 1 to 7, wherein the cancer is breast cancer (BC), ovarian cancer (OvCa) or pancreatic cancer (PaCa).

9. A kit for use in the method of any one of claims 1 to 8, the kit comprising means for determining the methylation status and/or expression level of said one or more genes, preferably comprising one or more primers for amplifying a fragment comprising a CpG site and/or for analyzing the product amplified by a primer pair, wherein said kit comprises instructions for carrying out said method, and optionally standards for a control or reference, preferably wherein the primer pair is a primer pair amplifying and/or comprising or consisting of the nucleotide sequence of S100P (NC_000004.12 Reference GRCh38 Primary Assembly, AC_000136.1 Alternate HuRef, N_018915.2 Alternate CHM1_1.1), SLC22A18 (NC_000011.10 Reference GRCh38 Primary Assembly, NG_011512.1 RefSeqGene, NT_187585.1 Reference GRCh38 ALT_REF_LOCI_1, AC_000143.1 Alternate HuRef, NC_018922.2 Alternate CHM1_1.1), DYRK4 (NC_000012.12 Reference GRCh38 Primary Assembly, AC_000144.1 Alternate HuRef, NC_018923.2 Alternate CHM1_1.1), FUT 7 (NC_000009.12 Reference GRCh38 Primary Assembly, NG_007527.1 RefSeqGene, AC_000141.1 Alternate HuRef, NC_018920.2 Alternate CHM1_1.1) or fragments thereof or its bisulfite-converted sequence, preferably wherein the primer or primer pair is selected from the group of primers comprising or consisting of the nucleotide sequence of SEQ ID NOs 5 and 6, 7 and 8, 9 and 10, and 11 and 12, respectively.

10. A device for diagnosing cancer, comprising
(a) an analyzing unit comprising
(i) a detection agent for determining the methylation status or expression level as defined in any one of claims 1 to 8 in a sample of a subject;
(ii) receptacle for a sample of a subject suspected to suffer from or being at risk of developing cancer; and optionally
(b) an evaluation unit comprising a data processor having tangibly embedded an algorithm for carrying out a comparison of the amount determined by the analyzing unit with a stored reference and which is capable of generating an output file containing a diagnosis established based on the said comparison.

11. A method for monitoring the therapy of a subj ect being treated against cancer, comprising the steps of:
(a) providing a biological sample from the subject being treated;
(b) determining the methylation status or expression level as defined in any one of claims 1 to 8,
wherein an increase of methylation and/or decrease of expression compared to a reference biological sample obtained from the subject before or at an earlier stage of the treatment indicate effectiveness of a treatment course undergoing by the subject

12. An anti-cancer agent for use in the prevention, amelioration or treatment of cancer in a patient
(a) at an early stage or before the onset of the cancer, wherein the patient is **characterized by** a decreased methylation status and/or increased expression of one or more genes or proteins selected from the group consisting of S100P, SLC22A18, DYRK4, and FUT7 as compared to a healthy subject or reference value, preferably wherein the patient has been diagnosed by the method of any one of claims 1 to 8, preferably wherein
(i) the cancer is BC and
(ii) the patient is younger than 50 years and/or does not show micro-metastasis in lymph nodes, or
(b) wherein the dose of the agent is adjusted in accordance with
(i) the diagnosis of the method of any one of claims 1 to 8; or
(ii) the progression or regression of the cancer as assessed by the method of claim 11, preferably wherein the anti-cancer agent is selected from group consisting of Herceptin (Trastuzumab), Avastin (Bevacizumab), and the thyrosine kinase inhibitiors such as Lapatinib, Sorafinib oder Sunitini; Epirubicin, Doxorubicin, Cyclophosphamid, Taxol, Taxotere, Carboplatin, GnRH-analoga, estrogen receptor blocker such as Tamoxifen and Fulvestrant, aromatase inhibitors such as Arimidex, Femara, and Aromasin; Perjeta (Pertuzumab)

13. A method of classifying cancer or determining the risk of developing cancer in a subject, comprising:
(a) providing a sample from a subject,
(b) detecting the presence or absence of DNA methylation and/or expression level in one or more genes to generate a methylation profile and/or expression profile for said subject, wherein the one or more genes is selected from the group consisting of S100P (S100 calcium binding protein P), SLC22A18 (Solute carrier family 22, member 18), DYRK4 (dual-specificity tyrosine-(Y)-phosphorylation regulated kinase 4) and FUT7 (fucosyltransferase 7); and
(c) comparing said methylation profile and/or expression profile to one or more standard methylation profiles and/or expression profile, wherein said standard methylation profiles and/or expression profile are selected from the group consisting of methylation profiles and/or expression profile of non-cancerous samples and methylation profiles and/or expression profile of cancerous samples, preferably wherein said sample is a sample of peripheral blood and/or the expression level is determined by measuring the mRNA and/or protein level.

14. A non-transient computer readable storage medium, comprising executable instructions for detecting cancer or a susceptibility of developing cancer, the executable instructions configured to:
(a) receive data characterizing the intensity of DNA methylation and/or expression level in one or more gene loci as defined in any one of claims 1 to 8;
(b) compare the intensity of DNA methylation and/or expression level to a model; and calculate a likelihood of cancer.

15. A method of dosing an anti-cancer agent or an agent for use in amelioration of symptoms associated with the development of cancer in a subject comprising the method of any one of claims 1 to 8, 11 or 13, and preparing a pharmaceutical composition comprising an effective amount of and/or dosage regime for use of the agent in the treatment of the subject.

16. Use of one or more genes selected from the group consisting of S100P, SLC22A18, DYRK, and FUT7 or an expression product of any one thereof as a biomarker for the early detection and/or classification of cancer.
